# EUROPEAN PATENT APPLICATION

(11) **EP 1 607 486 A1**
(43) Date of publication of application: **21.12.2005**
(21) Application number: 05012933.7
(22) Date of filing: 15.06.2005
(51) Int. Cl.: C12Q 1/68

(54) **Method of stress evaluation**

(30) Priority: 15.06.2004 JP 2004177147
(71) Applicant: Rokutan, Kazuhito, c/o Department of Stress Science, Inst. of Health Biosciences, The University of Tokushima Graduate Scool, Tokushima-shi, Tokushima 770-8503 (JP); Hitachi Ltd., Tokyo 100-8280 (JP)
(72) Inventor: Rokutan, Kazuhito, Unv. Tokushima Graduate School, Tokushima-shi Tokushima 770-8503 (JP); Morita, Kyoto, c/o Unv. Tokushima Graduate School, Tokushima-shi Tokushima 770-8503 (JP); Kamihara, Kumiko, Chiyoda-ku Tokyo 100-8220 (JP); Saito, Toshiro, Chiyoda-ku Tokyo 100-8220 (JP)
(74) Representative: Strehl Schübel-Hopf & Partner

(57) **Abstract**

This invention relates to a simple method for evaluating stress of a normal healthy subject with high accuracy is provided. In this method, the expression profile of specific genes that serve as stress marker genes is analyzed, and stress of the subject is evaluated based on the analysis results.

## Description

### CLAIM OF PRIORITY

The present application claims priority from Japanese application JP 2004-177147 filed on June 15, 2004, the content of which is hereby incorporated by reference into this application.

### FIELD OF THE INVENTION

The present invention relates to a method of stress evaluation. More particularly, the present invention relates to a method of stress evaluation for a subject based on expression profiles of specific genes present in m-RNA of peripheral blood from the subject.

### BACKGROUND OF THE INVENTION

The concept of stress is "a reaction to recover from a disturbed state induced by stress stimulation that is an external force imposed on a living body" and a reaction important for protecting the body similarly to immune reaction and inflammation reaction. In an ordinary stress reaction, when stress stimulus is externally imposed to a body, various reactions take place in individual tissues in response to a signal generated from the central nervous system. When these reactions proceed to a certain degree, a feed back mechanism to suppress them usually begins to work. However, an excessive or unnecessary stress reaction due to a certain cause leads to various influences on the living body.

It has been empirically known for a long time from clinical observations that mental and physical stress is involved in various diseases. For example, diseases that are caused or promoted by stress include ischemic heart disease, hypertension, stress ulcer, gastrointestinal dysfunctions, eating disorders, chronic headache, and the like. Further, social problems such as NEET (Not in Employment, Education, or Training) and medical problems such as alcoholism disorders are also deeply associated with stress. Because of an increase in social stress nowadays, great attention is being focused on establishment of a system of stress evaluation with the aim of obviating these disorders.

Although life events that are high in mental stress stimulation generally include death of a close relative, disease, examination, change of abode, change of job, childbirth, and the like, these stimuli do not necessarily lead to diseases. The reason is that a serious burden to an individual is not necessarily so serious to another individual even if they undergo similar stress stimulation. In other words, an effect that stress stimulation imposes on a living body is dependent not only on the strength of the stress stimulation but also vulnerability of the individual subject to the stress.

The individual vulnerability is determined by family history, physical health condition, growth history, life history, social adaptability, personality inclination, and the like. Among them, when the personality inclination is classified into type A represented by a personality that is characterized by the sense of urgency over time, diligence, appetite for work, hostility, and aggression, and type B if that is not the case, there is data obtained from an epidemiological survey that the incidence rates of hypertension and ischemic heart disease are two to three fold higher in persons classified as the type A. Recently, however, there are not a few cases that cannot be judged in this way.

At the beginning of the 1990s, physiological indexes targeted for a series of research on stress evaluation included eyeblink, blood pressure, heart rate variability, respiration, perspiration, and the like. These studies aimed at direct measurement of stress-related components, and therefore did not bring about a method for accurately evaluating stress arising from complex factors. Subsequently, a method that employs subjective evaluation together with prolonged measurement of physiological indexes, a method by estimation of autonomic nervous system function, and the like were introduced as a method of stress evaluation. However, individual differences in stress levels of subjects, kinds of stress stimuli, kinds of tasks, reactions to the tasks, ways of coping with the tasks, and the like are large, thus leaving a problem that "stress evaluation should be personally and continuously performed by a simple method".

On the other hand, an oligonucleotide array that allows symptoms and disorders of stress and the like to be conveniently evaluated by gene expression levels is disclosed in JP-A No. 340917/2002. This array has a feature that allows the test result to be understood at a glance by arranging genes on a substrate based on their functions and their mutual interrelationships. That is, JP-A No. 340917/2002 does not provide specific genes that should be utilized as indexes for stress evaluation, and no appropriate objective test method of stress evaluation is yet available under the current state of the art.

### SUMMARY OF THE INVENTION

An object of the present invention is to provide a simple method for evaluating stress of normal healthy subjects objectively with high accuracy. Particularly, an object of the present invention is to provide a highly reproducible and reliable array for stress evaluation in which the number of DNA fragments immobilized on the array is minimized by specifying genes essential for evaluation of stress intensity.

The present inventors have focused on peripheral blood leukocytes that can be easily obtained as specimens as well as express many receptors for stress-related factors in order to evaluate stress reactions triggered by stress stimuli. They have exhaustively analyzed the expression patterns of mRNAs of 1,500 genes associated with stress responses and thereby found a method capable of evaluating stress with high accuracy to complete the present invention.

That is, the present invention provides a method of stress evaluation characterized in that expression levels of genes including ten genes listed in Table III are analyzed using mRNAs from peripheral blood of a subject and stress of the subject is evaluated based on the analysis result.

In an embodiment of the present invention, the method of stress evaluation is characterized in that expression levels of genes including the ten genes listed in Table III and/or eleven genes listed in Table IV are analyzed using mRNAs from peripheral blood of a subject and stress of the subject is evaluated based on the analysis result.

In another embodiment of the present invention, the method of stress evaluation in which expression levels of genes selected from those listed in Table I or Table II are analyzed using mRNAs from peripheral blood of a subject and stress of the subject is evaluated based on the analysis result is characterized in that the genes include at least ten genes listed in Table III.

In still another embodiment of the present invention, the method of stress evaluation in which expression levels of genes selected from those listed in Table I or Table II are analyzed using mRNAs from peripheral blood of a subject and stress of the subject is evaluated based on the analysis result is characterized in that the genes include at least ten genes listed in Table III and/or at least eleven genes listed in Table IV.

In the method of the present invention, the genes listed in Table I and Table II are genes specified for markers of stress evaluation. Further, the ten genes listed in Table III are particularly useful among the markers of stress evaluation as well as especially useful as marker genes for evaluation whose expression levels are upregulated by stress. Still further, the eleven genes listed in Table IV are especially useful as marker genes for evaluation whose expression levels are downregulated by stress.

In an embodiment of the method of the present invention, stress evaluation of a subject is carried out by performing comparative analysis of a gene expression profile of a universal RNA sample with that of the subject.

In another embodiment of the method of the present invention, stress evaluation of a subject is carried out by performing comparative analysis of gene expression profiles of the same subject in normal times and under stress load.

Although the method for analysis of gene expression used in the present invention is not particularly limited, a solid substrate comprising immobilized DNA such as DNA chip, DNA array, membrane filter, and capillary is preferred from the standpoint that a number of genes can be exhaustively analyzed at a time.

The solid substrate can be prepared by immobilizing each probe that hybridizes specifically to any one gene selected from the genes listed in Table I or Table II for detection of the respective genes on a solid substrate such as glass or nylon membrane. The present invention also provides such solid substrate for stress evaluation. The genes targeted for detection and immobilized on a solid substrate preferably include at least FRAT1, NFIL3, SCYB5, BTK, IL8RB, GRO1, CSF3R, LMNB1, HSPA6, and IFNGR2 that are shown in Table III and further GZMA, IL2RB, IGFBP7, CCNA2, PPP3CC, COX10, HSPA10, CDC16, TRAF5, RBBP7, and LIPA that are shown in Table IV.

Furthermore, the present invention provides a system of stress evaluation to perform the method of stress evaluation of the present invention that is characterized by being provided with means for performing comparative analysis between gene expression data of a subject and gene expression data of the subject obtained beforehand in normal times or that of a universal RNA sample.

In the present invention, stress of a normal healthy subject is evaluated by analyzing gene expression levels in peripheral leukocytes using a DNA chip while paying attention not only to individual genes but also to alteration of a balance in each of the nervous system, the endocrine system, and the immune system.

### BRIEF DESCRIPTION OF THE DRAWINGS

Figs. 1A to 1V show ratios of fluorescence intensity (Cy5/Cy3) of 519 genes targeted for analysis;
Fig. 1W is a colored chart showing results of cluster analysis for the 519 genes targeted for analysis;
Figs. 2A to 2C show the ratios of fluorescence intensity (Cy5/Cy3) of significantly upregulated genes by stress load;
Fig. 2D is a colored chart showing results of cluster analysis for the significantly upregulated genes by stress load;
Figs. 3A to 3C show the ratios of fluorescence intensity (Cy5/Cy3) of significantly downregulated genes by stress load;
Fig. 3D is a colored chart showing results of cluster analysis for the significantly downregulated genes by stress load;
Figs. 4A and 4B show the ratios of fluorescence intensity (Cy5/Cy3) before and after music appreciation of genes listed in Table I;
Fig. 4C is a colored chart showing results of cluster analysis for the genes listed in Table I;
Figs. 5A and 5B show the ratios of fluorescence intensity (Cy5/Cy3) before and after the music appreciation of genes listed in Table II;
Fig. 5C is a colored chart showing results of cluster analysis for the genes listed in Table II; and
Fig. 6 is a schematic diagram of the method of stress evaluation of the present invention.

### DETAILED DESCRIPTION OF THE INVENTION

### 1. Stress marker gene

The present inventors extracted RNA from the whole blood collected from normal healthy subjects who were confirmed not to be affected with a mental or somatic disease and exhaustively analyzed expression levels of 1,500 genes with the use of a DNA chip. Based on these results, stress marker genes were determined. DNA chip is constructed by immobilizing DNA fragments having nucleotide sequences corresponding to a number of genes on a support substrate such as glass and used to detect mRNA in a sample by hybridization. In place of DNA chip, other types of solid substrates (DNA array, capillary, membrane filter, etc.) or quantitative methods may be employed as long as analysis of gene expression can be exhaustively performed. It should be noted that the term "stress" in the present invention does not mean physical stress such as exercise or pain but mental stress such as strain, anxiety, or fear.

Specifically, students facing oral presentation of their master thesis were targeted, and ratios of expression levels of each gene immediately before, immediately after, and one day after the presentation were determined, respectively, with reference to the levels in normal times when samples were collected four weeks before the presentation. Genes (519) that showed a fluorescence intensity higher than 300 in all 30 data pairs consisting of 10 comparative data pairs of immediately before presentation/in normal times (A), 10 comparative data pairs of immediately after presentation/in normal times (B), and 10 comparative data pairs of one day after presentation/in normal times (C) were targeted for cluster analysis.

As a result of cluster analysis, it was confirmed that the expression levels immediately before presentation (A) and one day after presentation (C) did not significantly vary compared to the expression levels in normal times, but that the expression levels immediately after presentation (B) markedly varied. From these results, 73 genes that were significantly upregulated immediately after presentation (immediately after undergoing stress stimulation) compared with in normal times and immediately before presentation and returned to normal expression levels one day after presentation (Table I) and 93 genes that were significantly downregulated compared with in normal times and immediately before presentation and returned to normal expression levels one day after presentation (Table II) were selected for "stress marker genes" respectively.

Further, among the above marker genes, genes that show small p-values (P≤0.25) between immediately before presentation (A) and immediately after presentation (B) and between immediately after presentation (B) and one day after presentation (C) were extracted by t-tests between the two groups of A and B and of B and C. The geometric average of the p-value between A and B and the p-value between B and C determined for each of the genes was ranked, and top ten genes were selected as especially useful marker genes (Table III).

All of the ten genes selected in the above paragraph were genes upregulated by stress load. Next, as to downregulated genes by stress load, especially useful marker genes were also selected as follows. Genes that show small p-values (P≤0.25) between immediately before presentation (A) and immediately after presentation (B) or between immediately after presentation (B) and one day after presentation (C) were extracted by t-tests between the two groups of A and B and of B and C, followed by ranking as in the above paragraph. Then, top eleven genes were selected (Table IV).

Namely, the genes listed in Table I and Table II are marker genes specified for stress evaluation in the present invention. Among them, the ten genes listed in Table III are especially useful as marker genes for stress evaluation as well as especially useful as marker genes upregulated by stress. The eleven genes listed in Table IV are especially useful as marker genes downregulated by stress.

### 2. Characteristics of marker genes

The genes selected for stress marker genes mainly include apoptosis-related genes, ATPase-related genes, cell cycle-related genes, cytokine-related genes, heat shock protein-related genes, polymerase-related genes, GTP-binding protein-related genes, protein kinases, stress-responsive cytochrome c oxidase components of mitochondria, and oncogenes.

Mental stress stimulation induces adaptive responses by controlling functions of the autonomic nervous system and the endocrine system. In the analysis results of the present invention, upregulation of angiotensin II (AGTRL2) was observed after oral presentation of master thesis. On the other hand, downregulation of angiotensin I that is a precursor of angiotensin II was observed conversely. Angiotensin has a strong vasopressor activity and is pointed out to be related to mental disorders caused by stress. In addition, polymorphism analysis of ACE gene has also been carried out with respect to schizophrenia (Neuropsychobiology 2001; 44(1): 31-35).

It has already been known that stress modifies immune functions. It has been demonstrated that undergoing stress results in an increase of the number of leukocytes in peripheral blood, a decrease of the numbers of T cells, B cells, and helper T cells, a decrease in the activity of NK cells, and the like (PsychosomMed 1993; 55: 364-379), and these are also proposed to be used as biochemical stress indices. Since downregulation of T cell receptor (TRB, CD8B1) and B cell receptor (CD79B) was observed right after undergoing stress stimulation in the analysis results of the present invention, the stress evaluation according to the present invention seems to be appropriate.

Further, inflammatory cytokines such as interleukin (IL)-1, 6, and 8 exhibit a wide variety of physiological actions related to stress reactions, and their actions on the central nerve system lead to fever, drowsiness, loss of appetite, and the like. In the present analysis as well, mRNAs of IL-1 receptor (IL1R1, IL1R2), IL-13 receptor (IL13RA1), GM-CSF receptor (CSG2RA), IL-8 receptor (IL8RA, IL8RB), IL-2 receptor γ-chain (IL2RG), interferon-regulatory factor 2 (IRF2), interferon-inducible protein (IFITM1), interferon γ-inducible protein (IFITM3), interferon γ receptor (IFNGR2), and the like were upregulated. In contrast, mRNAs of IL-10 receptor (IL10RA), IL-11 receptor (IL11RA), IL-2 receptor β-chain (IL2RB), IL-13 receptor (IL13RA2), CX3C chemokine precursor (SCYD1), and the like were downregulated. Stress stimulation is known to have an influence on immune system, and variations in gene expression of many cytokine related genes were also observed in the present analysis results.

Differences in intensity of signals from various stimuli are controlled by a balance between activities of protein kinases and protein phosphatases. Accordingly, downregulation of mRNA expression of CD45 (RBBP4) is considered to be influential on immunological functions to a significant degree. Further, TAK1 (MAP3K7), that is present in the downstream of TGF-β and Toll-like receptors and serves as an important signal transduction molecule for these receptors, and β1-integrin (ITGB1) were both downregulated, and both of these returned to normal on the next day after being relieved from stress stimulation. From these results, mRNA expression pattern of regulatory factors for immune cell functions is considered to be highly useful for stress evaluation.

Similarly, relatively large variations in the heat shock protein (HSP) family that are induced by various environmental stresses and contribute to stress response and tolerance of cells were found in leukocytes from subjects who underwent stress stimulation. Upregulation of mRNA expression of MHC-associated HSP70-1 (HSPA1A), HSPA1L, and HSPA6 was observed. In contrast, the expression of a significant number of heat shock proteins such as cognate heat shock protein 70 HSC70 (HSPA10), HSP90α (HSPCA), chaperonin HSP60 (HSPD 1), and chaperonin 10 (HSPE 1) whose expression ought to have been essentially upregulated by stress was, however, found to be downregulated. This may be considered to be reflecting that a balance of stress reactions is lost due to excessive stress stimulation.

The heat shock protein HSP70 expressed by undergoing stress stimulation suppresses apoptosis. However, when it is expressed excessively, it has been reported that the activation of intracellular pathways leading to apoptosis such as c-Jun N-terminal kinase (JNK) and caspase is blocked (Mol. Cell. Biol. 1997; 17: 5317-5327).

According to the present analysis results, the expression of caspase related genes (APAF1, CFLAR) was upregulated in a sample immediately after undergoing stress stimulation, while the expression of apoptosis related genes (DAP3, CSE1L) that promote cell death was downregulated. Further, mRNA expression of glycogen synthase kinase 3β, APC protein, and the like involved in the signaling pathway of Wnt that is a cell growth promoting factor were found to be upregulated. In concurrence with this, upregulation of BCL3, RALB, VAV2, FOS, RAF1, ARHA, LYN, SKIL, and the like that are oncogenes were characteristically observed. Rb-related proteins (RBBP4, RBBP7) that are tumor suppressor genes were downregulated. The expression levels of any genes returned to levels in normal times when relieved from the stress stimulation. When these results are taken together, it can be inferred that there is a possibility that malignant transformation of cells may be developed or advanced by undergoing excessive stress stimulation.

On the other hand, mRNA expression of genes (CDKN2C, PCNA, CCNA, CDC10) involved in the cell cycle regulation, DNA polymerases (POLG2, POLE) involved in DNA replication, and the enzyme RNA polymerase I- and II-associated genes (POLRMT, POLR2B, RPA40, TCEB 1, TCEB1L) involved in transcription was found to be downregulated by stress stimulation, and subsequently returned to levels in normal times one day after being relieved from the stress stimulation.

As described in the foregoing, the selected marker genes include a significant number of genes that have already been predicted to be related to stress, although these marker genes partially include genes that have not yet been reported to be related to stress, indicating that the stress evaluation of the present invention is appropriate. Whether individual genes are known to have a relation to stress is not an important point of the present invention. The point of the present invention lies in selection of specific genes (i.e. marker genes) that show a characteristic expression profile reflecting a stress state and demonstration of the validity of stress evaluation with the use of the genes.

### 3. Method of stress evaluation and system of stress evaluation

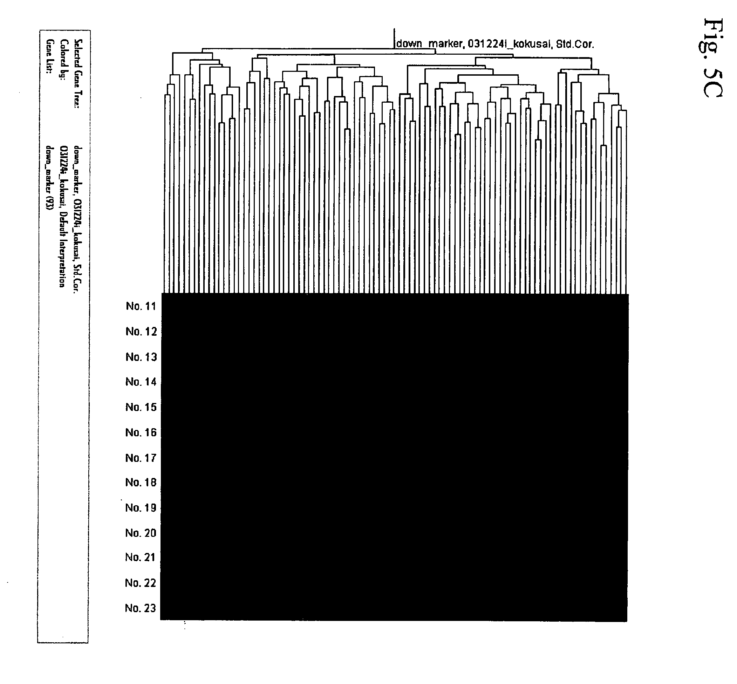

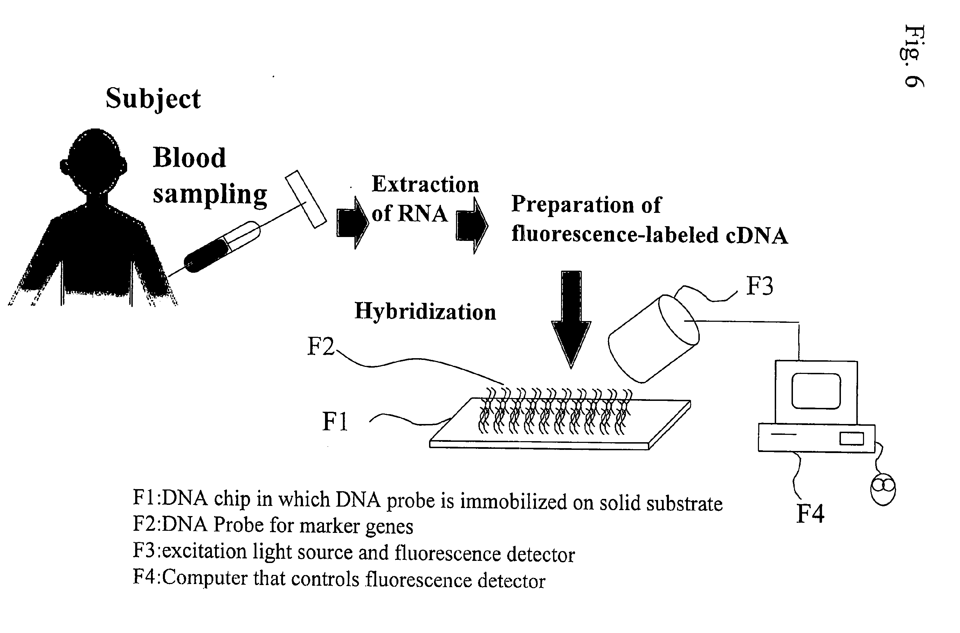

The present invention has been accomplished based on the afore-mentioned experimental results and relates to a method in which an expression profile of specific genes that serve as stress marker genes is analyzed with the use of mRNA from peripheral blood of a subject and stress of the subject is evaluated based on the analysis results. The schematic diagram of the method of stress evaluation of the present invention is shown in Fig. 6.

The method of gene expression analysis can employ any arbitrary method known in the art that includes nucleic acid hybridization using different types of solid substrates such as DNA array and membrane filter other than DNA chip, quantitative PCR such as RT-PCR and real-time PCR, Northern blotting, subtraction technique, differential display, differential hybridization, and the like. In particular, solid substrates such as DNA chip, DNA array, membrane filter, and capillary is preferred in view of an ability to analyze exhaustively a number of genes at a time.

The solid substrate used in the present invention can be prepared by immobilizing each probe that hybridizes specifically to any one gene selected from the genes listed in Table I or Table II for detection of the respective genes on a solid substrate such as glass or nylon membrane. The genes that are targeted for detection and immobilized preferably include at least ten genes listed in Table III, and more preferably include further the eleven genes listed in Table IV. The probes for detection of the genes may be designed as sequences complementary to highly specific region of the marker genes (for example, 3' UTR region) according to a known method, and are preferably from 100 to 1,500 nucleotides in length and more preferably from 200 to 1,100 nucleotides in length. The method of immobilizing the probes on a solid substrate is not particularly limited, and the synthesized probes may be spotted on the solid substrate or the probes may be synthesized on the solid substrate according to a known method.

Stress evaluation may be carried out by performing comparative analysis between gene expression profiles of a subject sample and a control sample with the use of a certain RNA sample, for example, a universal RNA sample as the control sample. The "universal RNA sample" is an RNA sample prepared by mixing total RNAs (or mRNAs) extracted from a group of different human tissues, and a standard RNA sample that covers most of the total expressed genes in human (preferably at least 90%). Although such a universal RNA sample can be prepared according to a conventional method, a commercially available universal RNA sample (for example, BD™ Premium RNA, human universal reference total RNA, Product of BD Biosciences Clontech) can preferably be used.

Alternatively, stress may be evaluated by performing comparative analysis of gene expression profiles of the same subject between in normal times and under stress load.

The method of data analysis is not limited to cluster analysis, and an arbitrary analysis method known in the art such as machine-learning algorithm with Support Vector Machine and the like can be used.

Once gene expression data of a subject in normal times and that of a universal RNA sample are accumulated in a database, stress state of the subject can be easily evaluated at any time simply by extracting the data of the same subject in normal times from the database. The present invention also provides such system of stress evaluation.

The method of stress evaluation of the present invention does not need any special cooperation of a subject and can be performed by analysis with 5 ml of blood obtained by ordinary blood collection, and therefore, it is a noninvasive, simple, and routinely performable evaluation method. The present method that allows biofunctions to be ascertained comprehensively from expression levels of numerous RNAs is, in principle, more appropriate as an evaluation method for complicated stress that involves mind and body, compared with a conventional method that measures limited factors.

### EXAMPLES

Hereinafter, the present invention will be explained in more detail by means of the following examples. However, the present invention is not limited to these examples.

### Example 1

Students facing oral presentation of master thesis were targeted, and mRNA expression levels immediately before, immediately after, and one day after the presentation were compared using a sample four weeks before the presentation as the reference in normal times.

### 1. Test method

Target subjects were those who provided informed consent to participate in the research for developing the present diagnostic method among students facing oral presentation of master thesis in the University of Tokushima, Faculty of Medicine. The present research was approved by the Ethics Committee of Tokushima University Hospital. Ten healthy target subjects consisted of two male and eight female students, and their ages ranged from 24 to 27 years (average; 24.9 years).

Five ml of blood was collected from each of the ten subjects at rest from the elbow vein at fasting time between 10 a.m. and 1 p.m. by a physician or a nurse. The collection of blood was carried out one to four weeks before, immediately before, immediately after, and one day after oral presentation of master thesis. Total RNA was extracted from each collected blood using PAXgene Blood RNA System (Product of Qiagen Inc.). The yield of the total RNA ranged from 5 to 15 micrograms.

Five micrograms of RNA was taken out from the total RNA extracted from each subject and subjected to annealing with an oligo(dT) 24 primer connected to a T7 promotor sequence to synthesize a first strand DNA. Then, with the use of this first strand DNA as a template, a second strand DNA having the T7 promotor sequence was synthesized. Finally, with the use of the second strand DNA as a template, RNA synthesis was carried out using T7 RNA polymerase. To six micrograms of the synthesized RNA were annealed random hexamers, followed by subjecting to reverse transcriptase reaction to synthesize a fluorescently labeled cDNA by allowing Cy5-dCTP to be incorporated into the strand. As to the reference sample collected one to four weeks before the presentation, cDNA was synthesized using Cy3 as the fluorescent label.

Equal amounts of the two kinds of cDNAs to be analyzed for comparison were mixed together and then applied onto a DNA chip (HITACHI DNA CHIP (for analyzing drug response), Hitachi Co., Ltd.) for hybridization at 62 degrees C for 12 hours. After washing, the fluorescence intensity of each spot was measured with a scanner (ScanArray 5000, product of GSI Lumonics Inc.). The ratio of expression levels of each gene was determined between respective sample pair of 10 pairs of samples immediately before presentation and four weeks before the presentation (A), 10 pairs of samples immediately after the presentation and four weeks before the presentation (B), and 10 pairs of samples one day after the presentation and four weeks before the presentation (C).

### 2. Results

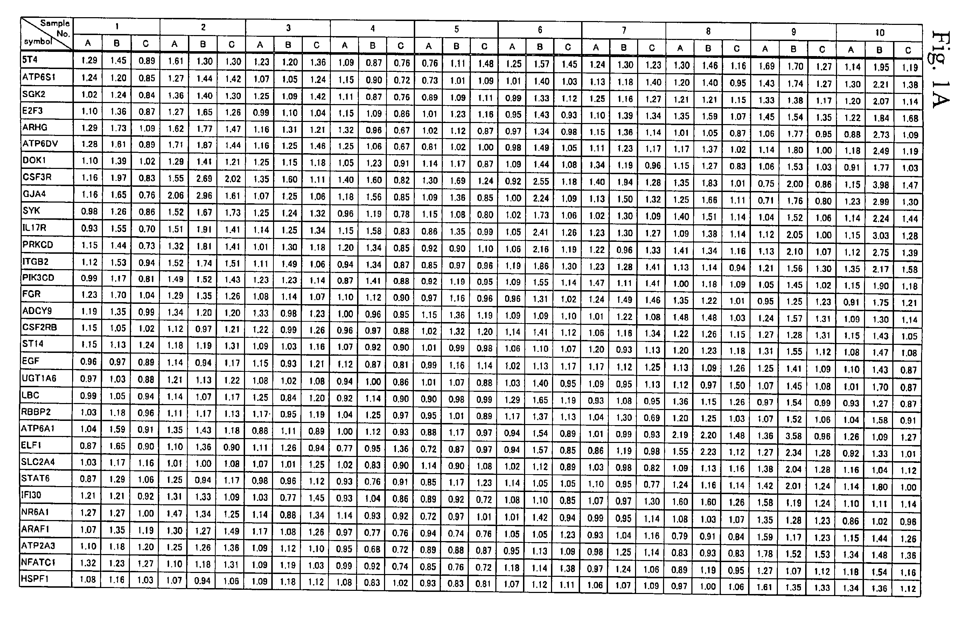

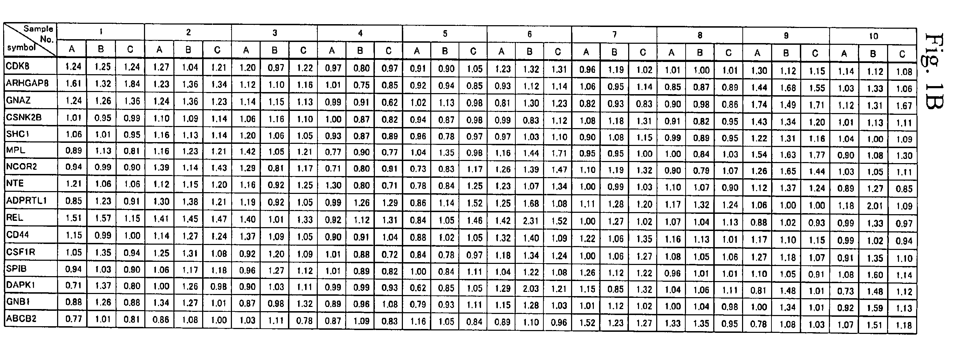

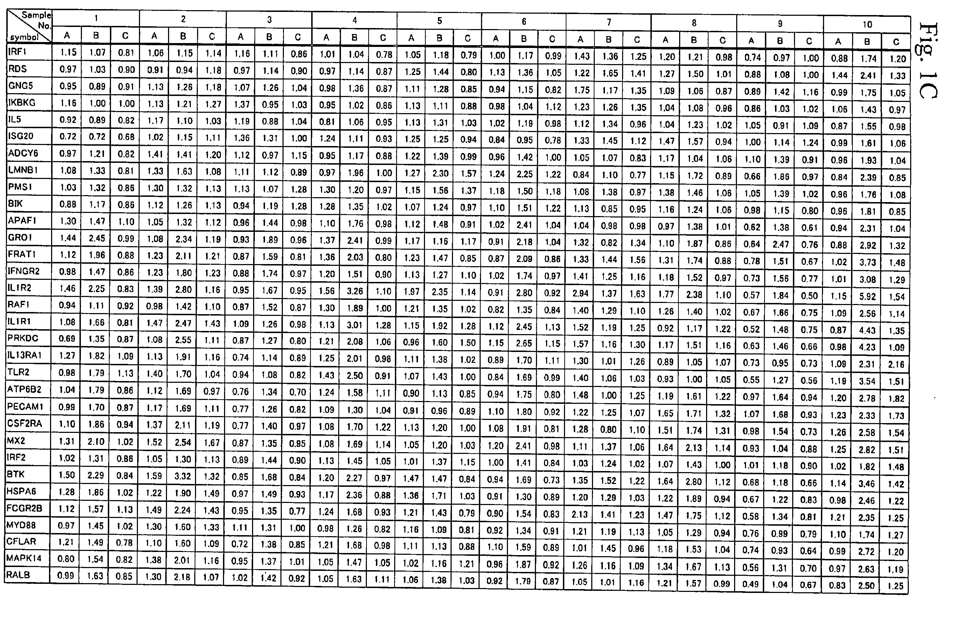

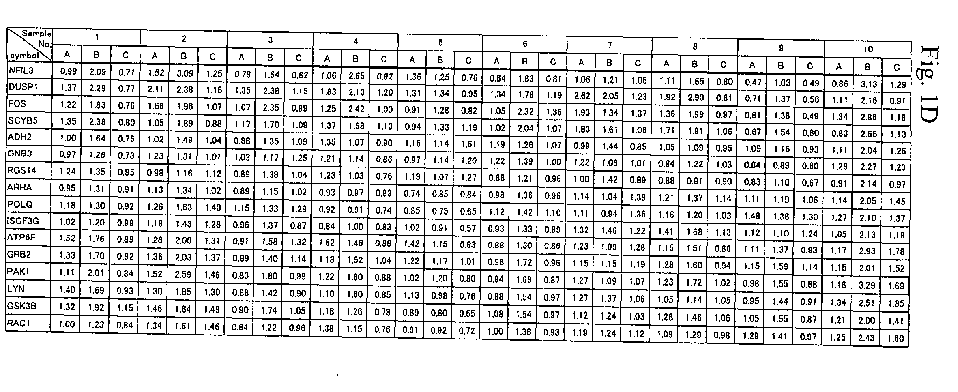

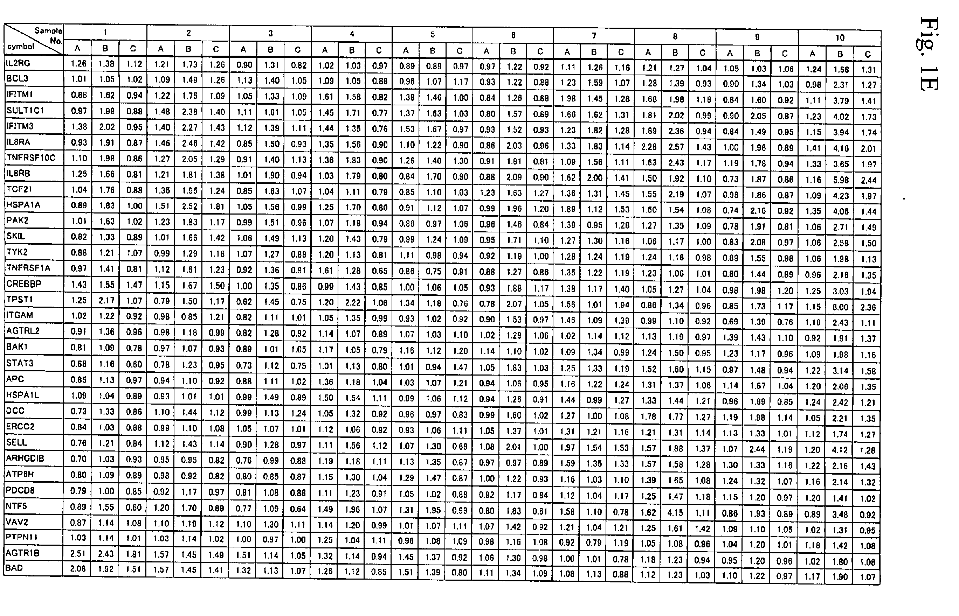

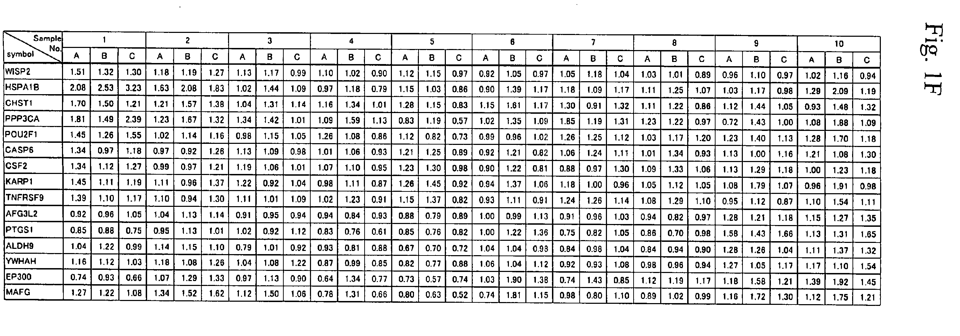

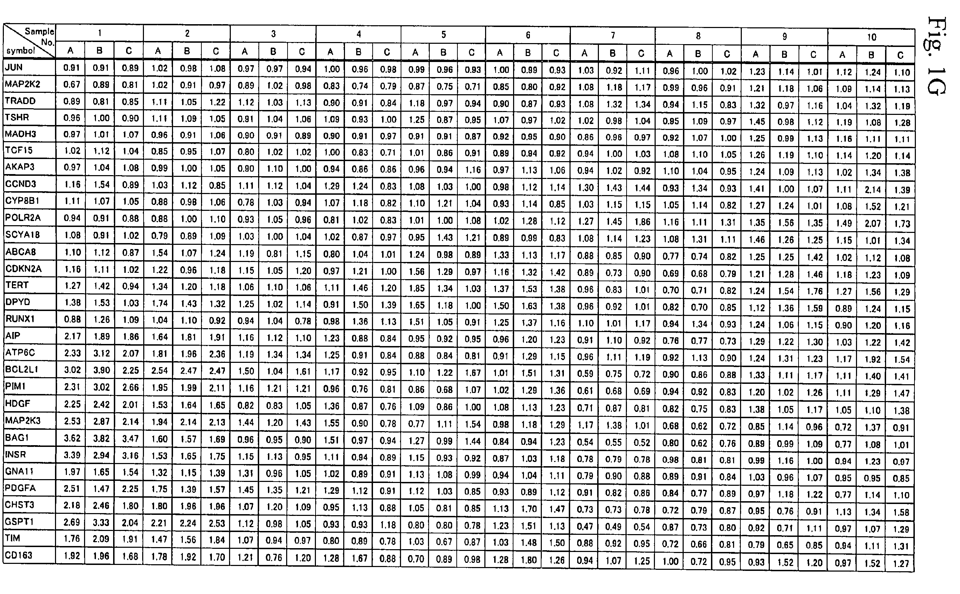

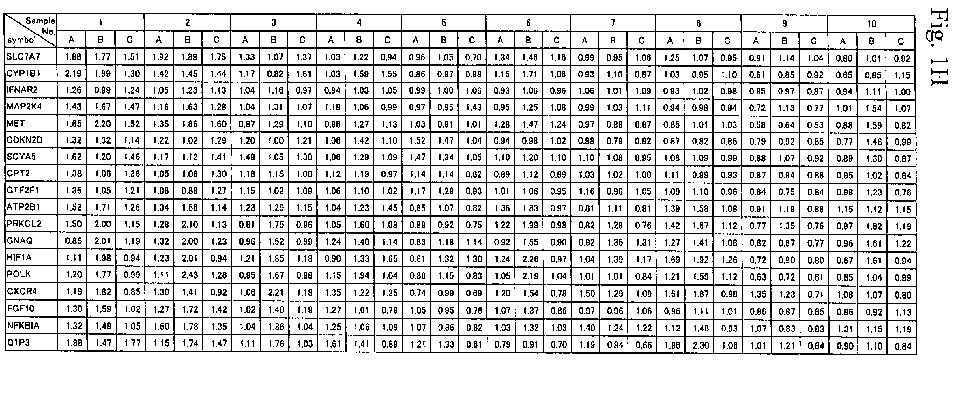

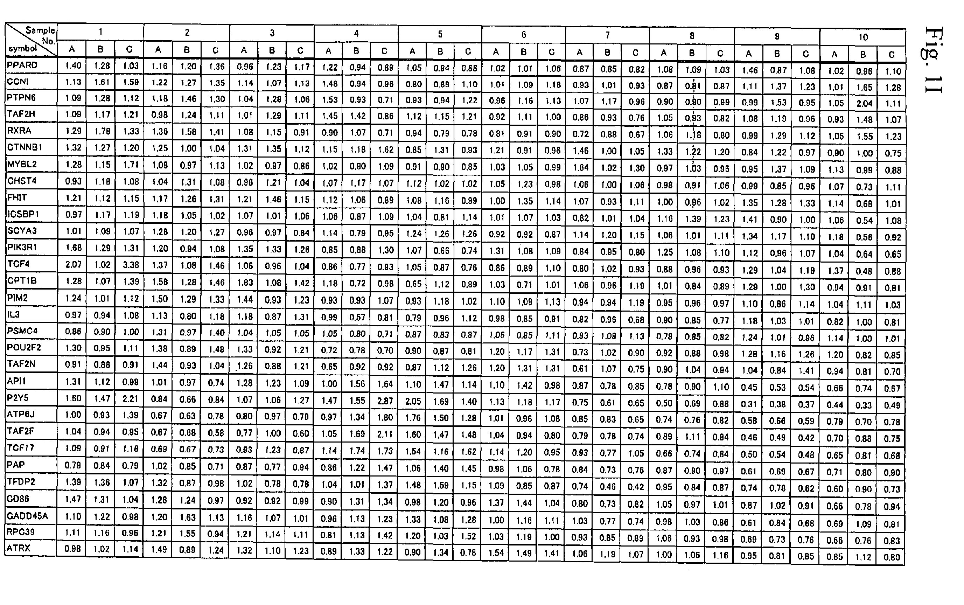

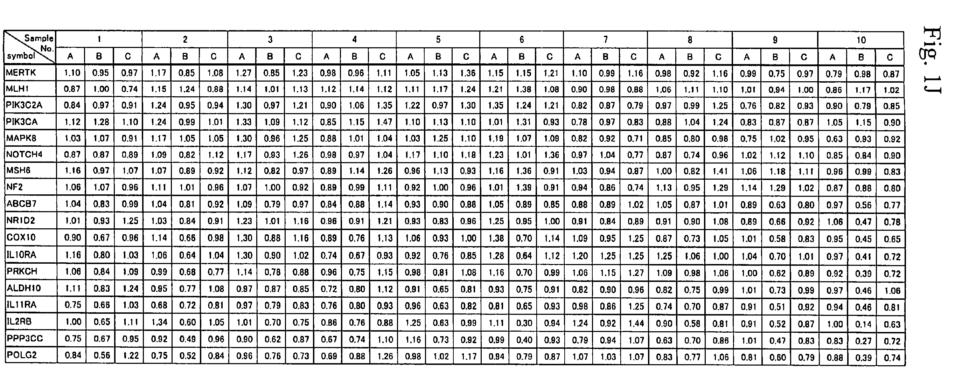

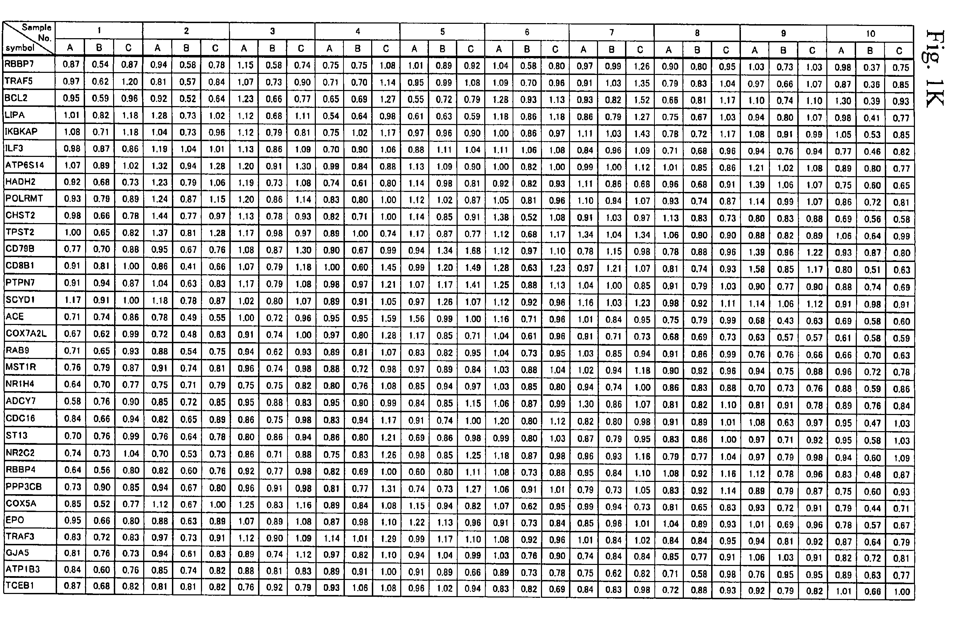

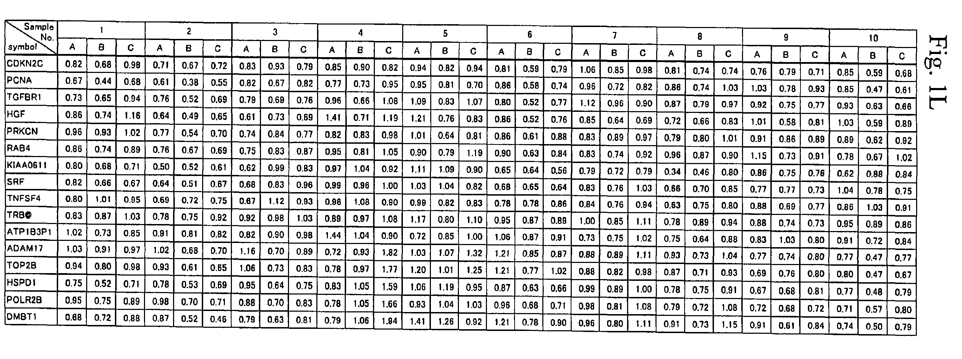

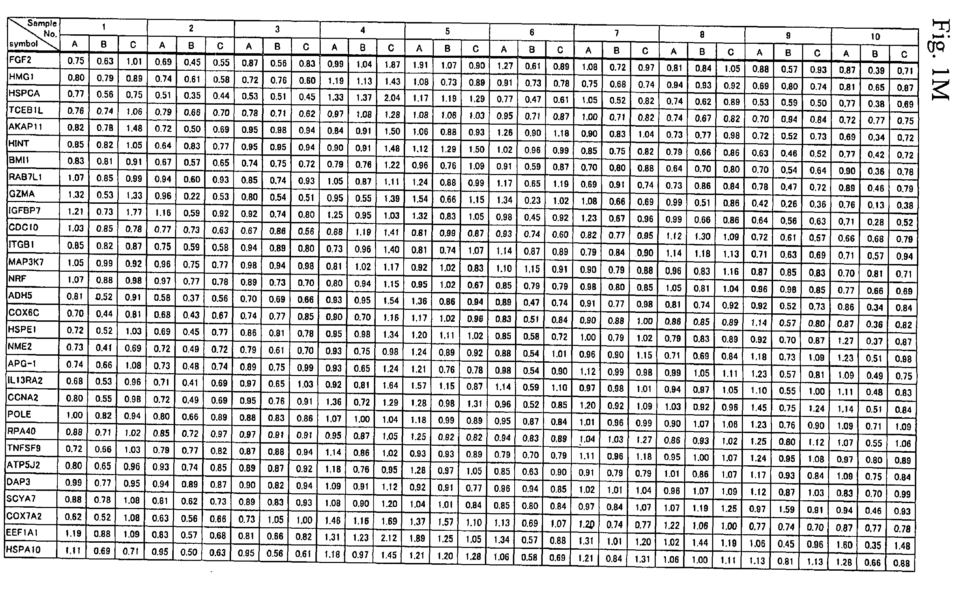

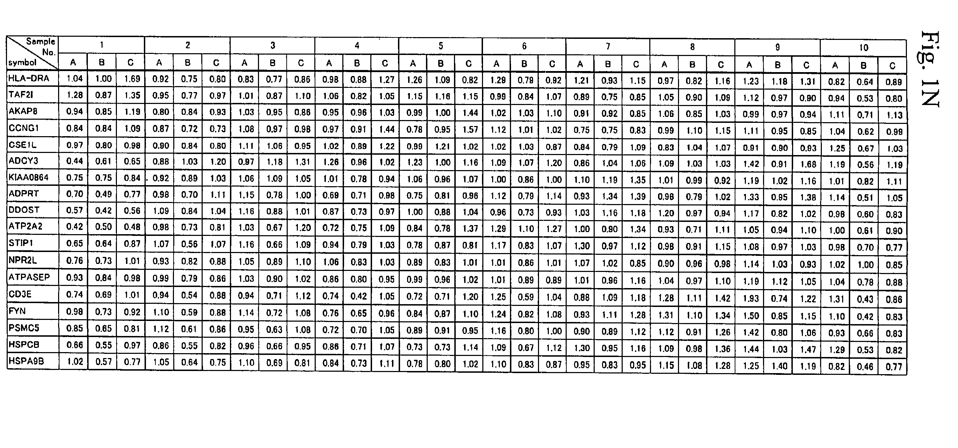

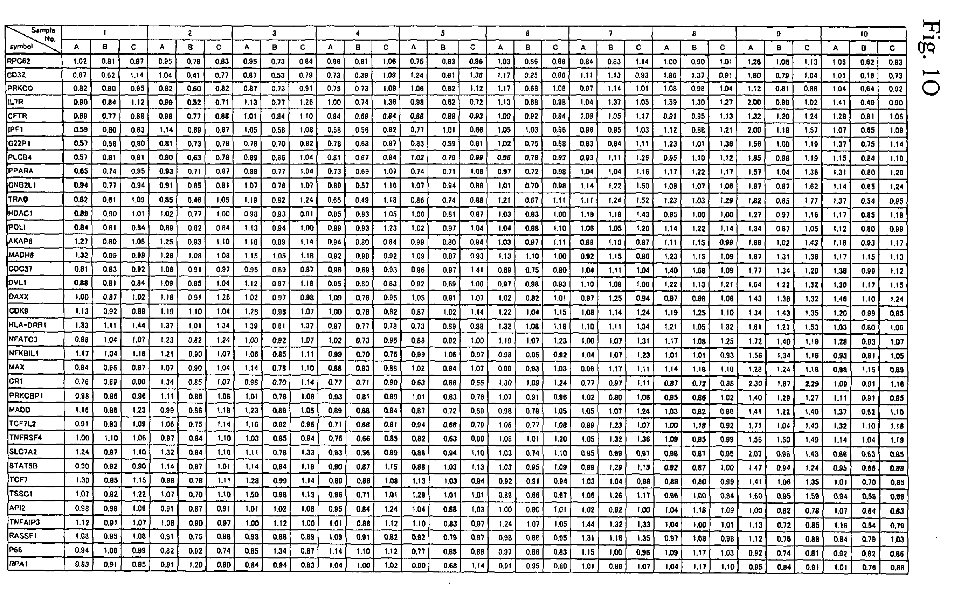

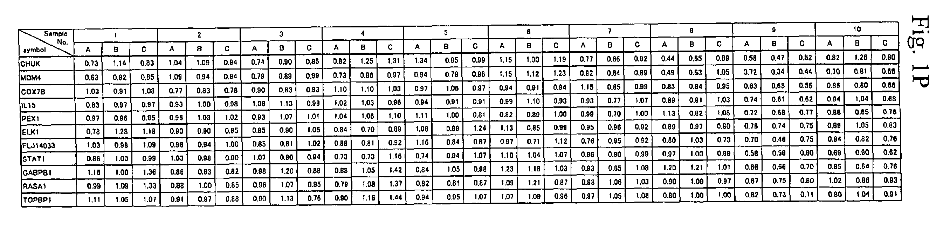

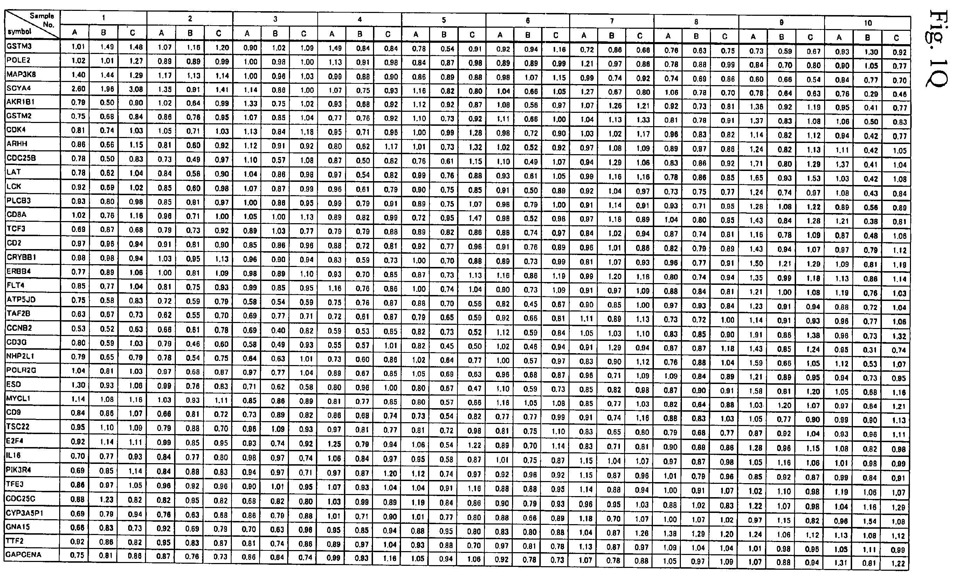

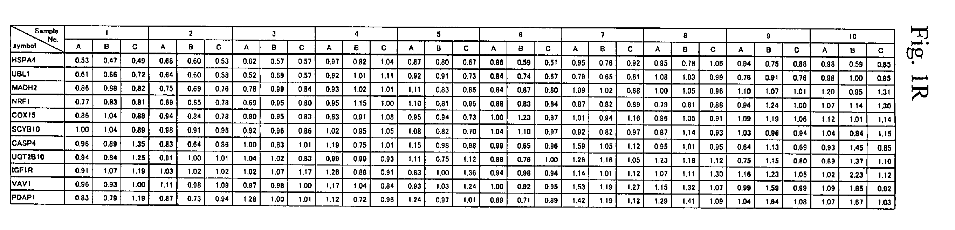

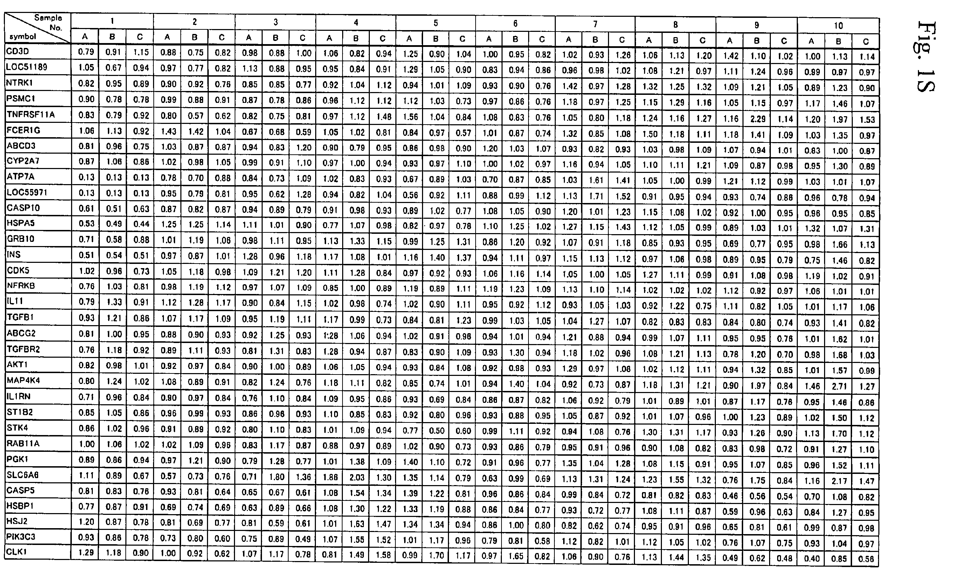

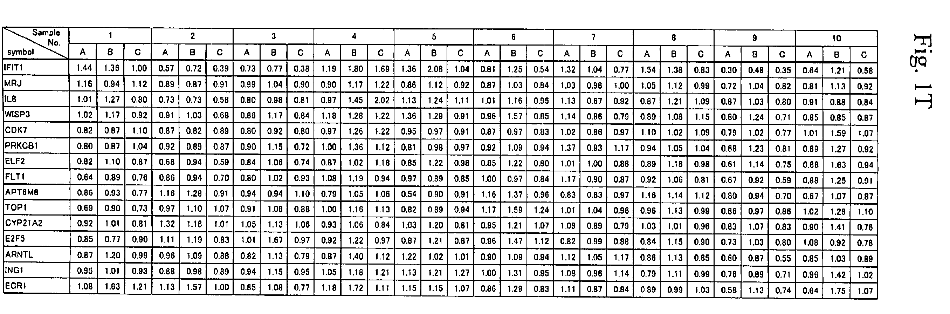

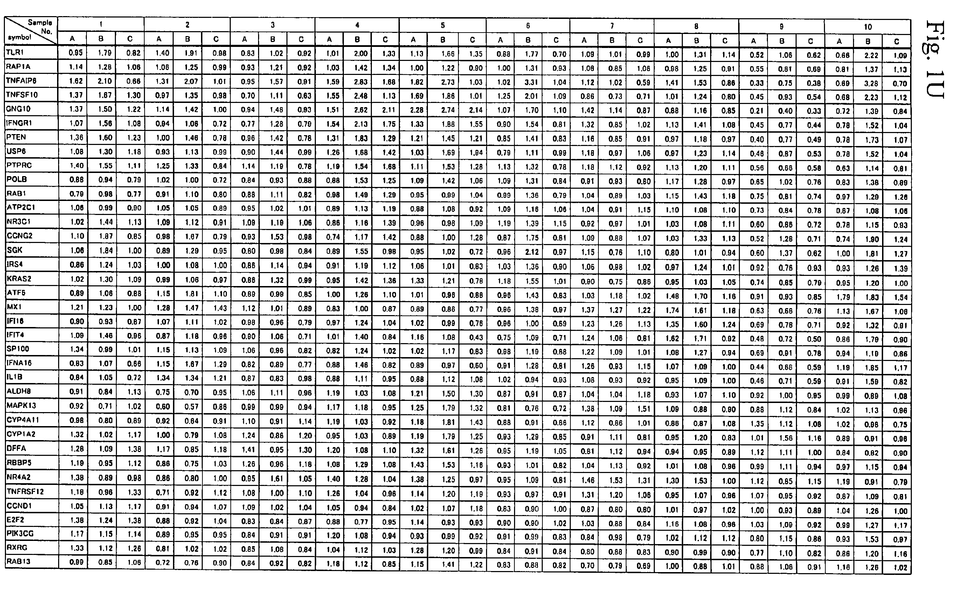

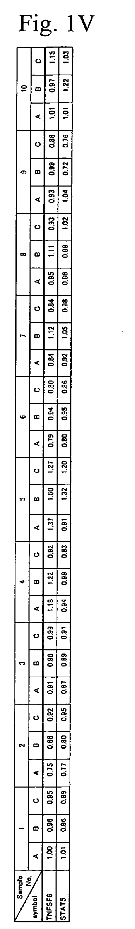

Genes (519) having fluorescence intensity higher than 300 were chosen from among all of the 30 data pairs, and a cluster analysis to group these genes was performed. Fig. 1 shows the results of expression analysis of the 519 genes. Figs. 1A to 1V show ratios of fluorescence intensities of each gene (Cy5/Cy3), and Fig. 1W is a colored chart showing the results of the cluster analysis. In these figures, the sample numbers (1 to 10) correspond to subject numbers, respectively, and the experiment number A represents comparison between immediately before oral presentation of master thesis (Cy5) and four weeks before the presentation (in normal times)(Cy3) of the same subject, the experiment number B represents comparison between immediately after the presentation (Cy5) and in normal times (Cy3) of the same subject, and the experiment number C represents comparison between one day after the presentation (Cy5) and in normal times (Cy3) of the same subject.

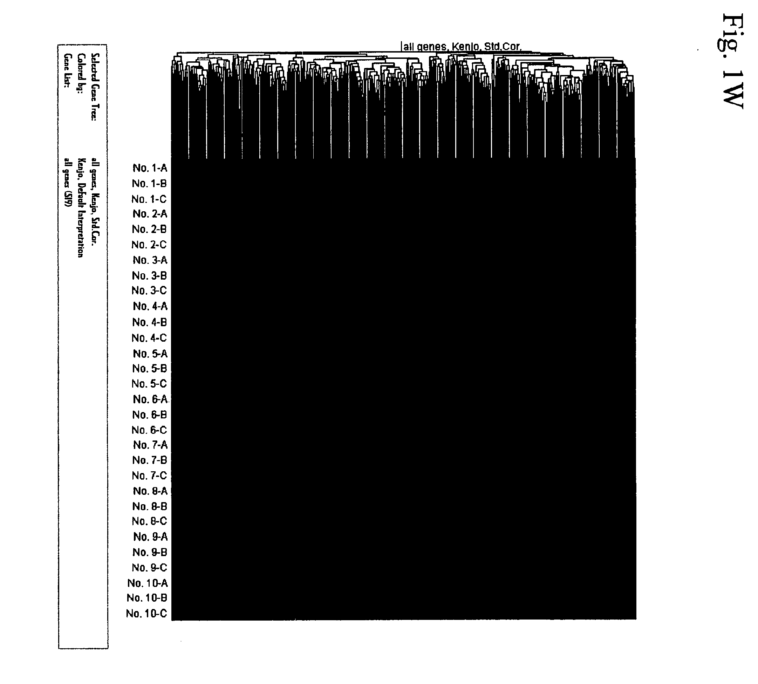

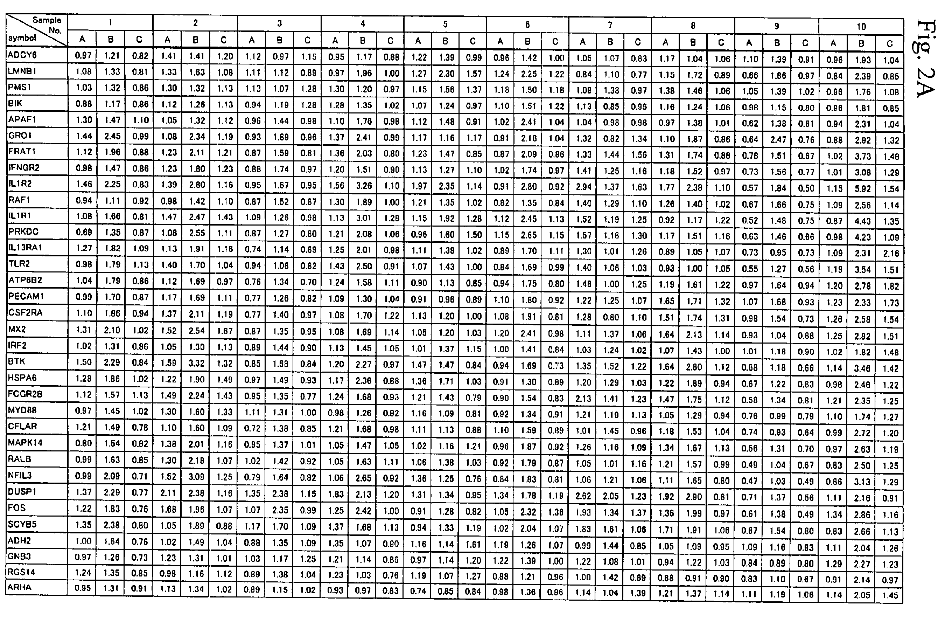

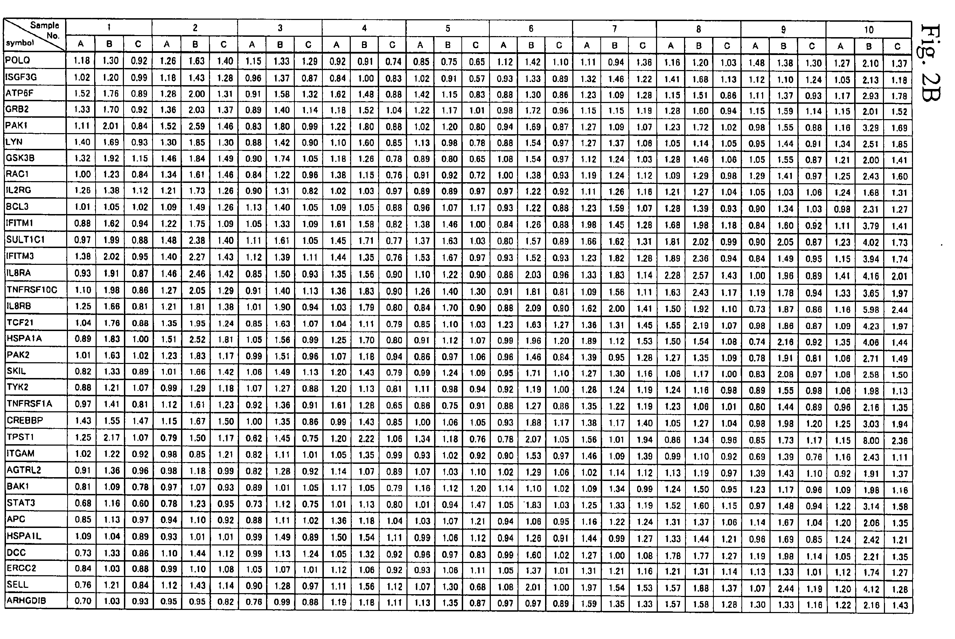

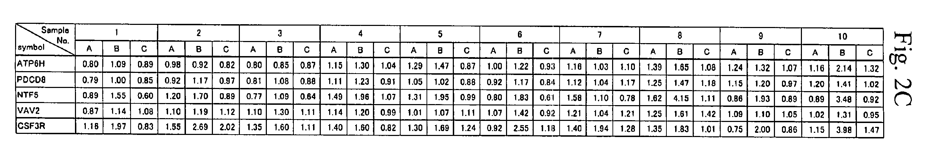

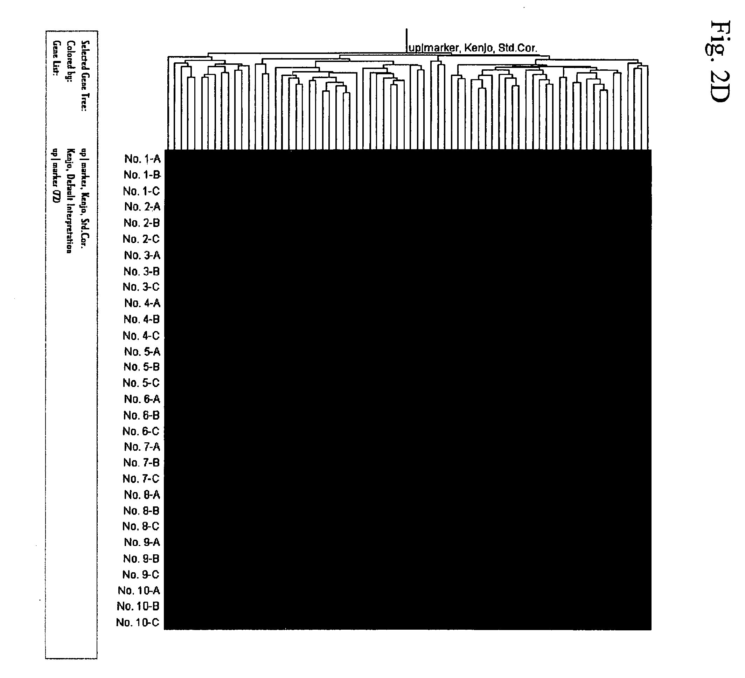

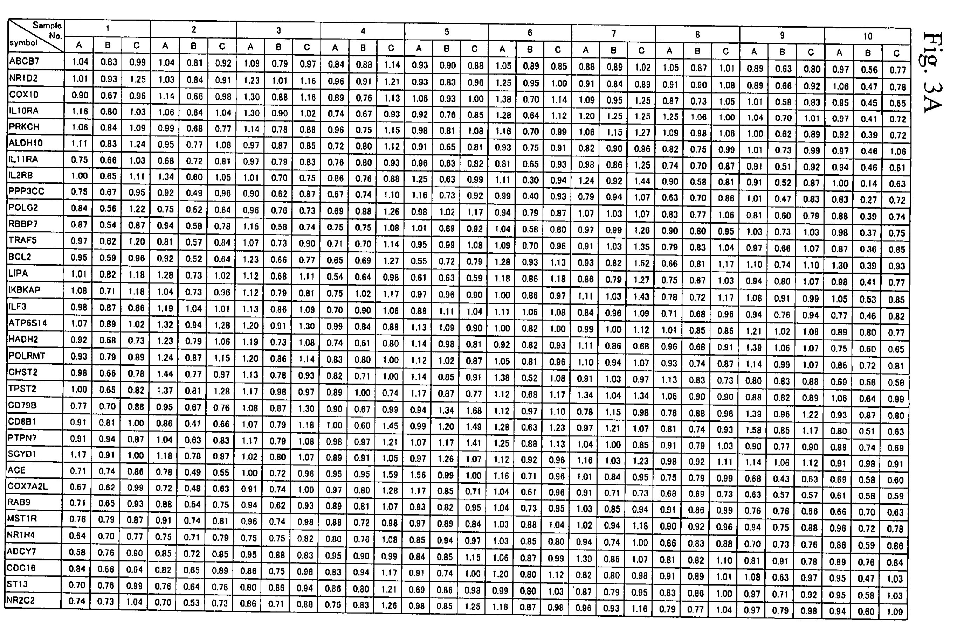

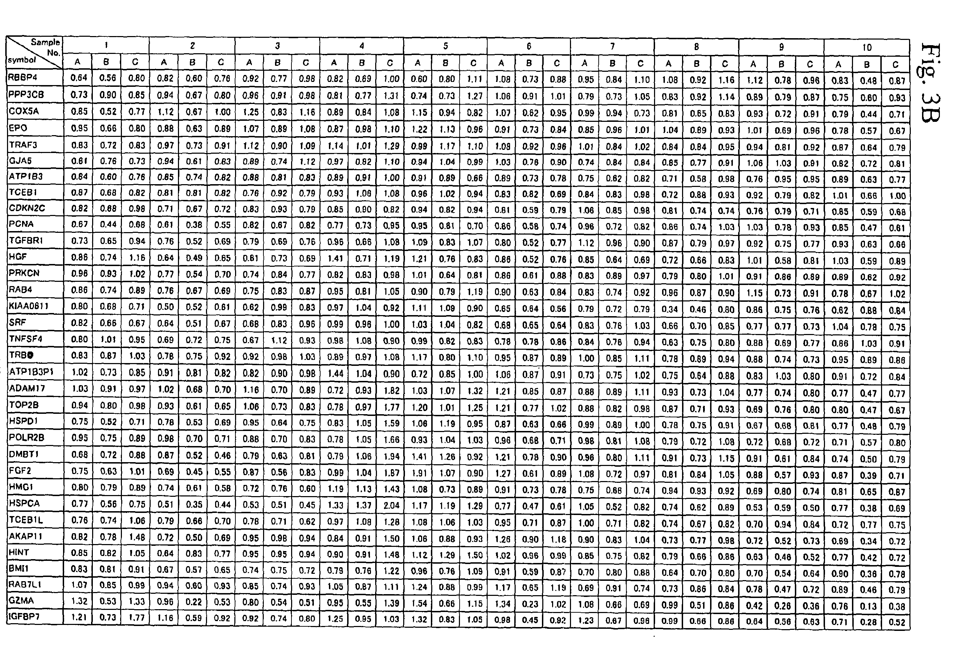

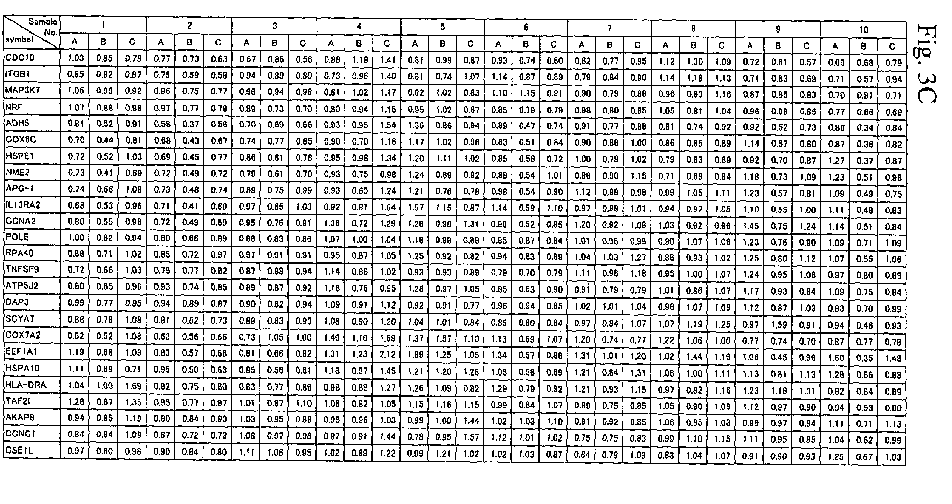

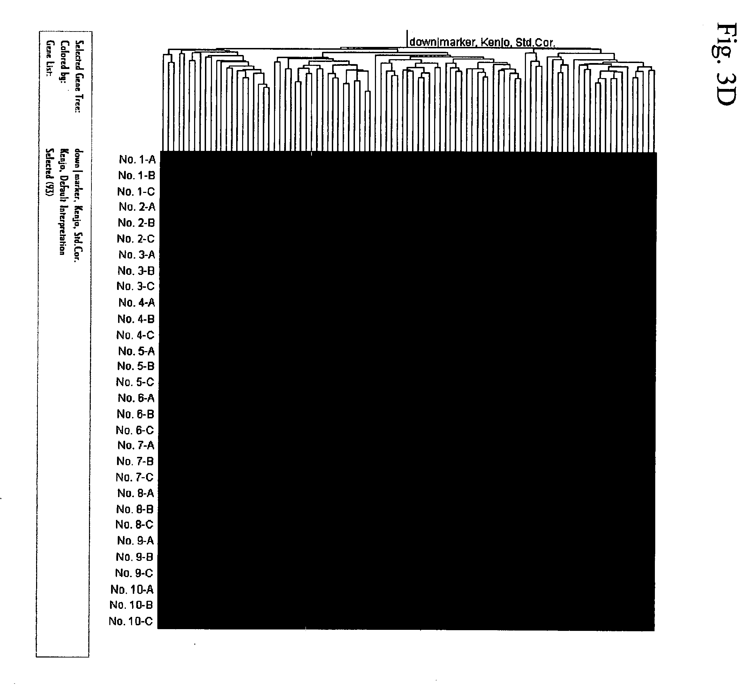

As the results of the cluster analysis, it was found that the genes shown in Fig. 2 had a tendency to be upregulated immediately after the presentation (immediately after undergoing stress stimulation) compared to in normal times and immediately before the presentation and to return to expression levels in normal times one day after the presentation. On the other hand, it was found that the genes shown in Fig. 3 had a tendency to be downregulated immediately after the presentation compared to in normal times and immediately before the presentation and to return to expression levels in normal times one day after the presentation. These specific genes represent genes that show marked variations in gene expression due to stress load and can be regarded as useful for marker genes to evaluate temporary mental stress. In other words, the time when the expression levels of these genes are varied compared to those in normal times is likely to be in a condition that stress stimulation is also felt physically as stress.

The genes shown in Figs. 2A to 2C and Figs. 3A to 3C that may serve as stress markers are summarized in Table I and Table II. Table I represents significantly upregulated genes (73 genes) under stress load compared to in normal times, and Table II represents significantly downregulated genes (93 genes) under stress load compared to in normal times.

**Table I:**

| Significantly Upregulated Genes by Stress Load Test | | | | |
|---|---|---|---|---|
| **Symbol** | **Name** | **Category** | **Pathway** | **GenBank** |
| ADH2 | Human class I alcohol dehydrogenase (ADH2) beta-1 subunit mRNA | ADH | | M21692 |
| AGTRL2 | Homo sapiens angiotensin receptor-like 2 (AGTRL2) | angiotensin | | NM_005162 |
| BAK1 | Human bcl2 homologous antagonist/killer (BAK) | Appoptosis | | U23765 |
| APAF1 | Homo sapiens apoptotic protease activating factor 1 (Apaf-1) mRNA, complete cds | Appoptosis, Signal | Mitochondria, D4GDI | AF013263 |
| CFLAR | Homo sapiens Casper mRNA; CASP8 and FADD-like apoptosis regulator; I-FLICE | Appoptosis, Signal | FAS | AF010127 |
| CREBBP | Human CREB-binding protein (CBP) mRNA, complete cds | ATF/CREB | TGFbeta | U47741 |
| ATP6B2 | ATPase, H+ transporting, lysosomal (vacuolar proton pump), beta polypeptide, 56/58kD, isoform 2 | ATPase | | L35249 |
| ATP6F | ATPase, H+ transporting, lysosomal (vacuolar proton pump) 21kD | ATPase | | D89052 |
| ATP6H | ATPase, H+ transporting, lysosomal (vacuolar proton pump) 9kD | ATPase | | Y15286 |
| IL1R2 | H.sapiens IL-1R2 mRNA for type interleukin-1 receptor, (cell line CB23). | II Cytokine | | X59770 |
| IL13RA1 | H.sapiens mRNA for IL13 receptor alpha-1 chain | Cytokine | | Y09328 |
| CSF2RA | Human GM-CSF receptor (GM-CSF receptor) mRNA, complete cds | Cytokine | | M73832 |
| MX2 | Human interferon-induced cellular resistance mediator protein (MxB) mRNA | Cytokine | | M30818 |
| IRF2 | Human mRNA for interferon regulatory factor-2 (IRF-2). | Cytokine | | X15949 |
| NFIL3 | Human bZIP protein NF-IL3A (IL3BP1) mRNA, complete cds | Cytokine | | U26173 |
| IFITM1 | Human interferon-inducible protein 9-27 mRNA, complete cds | Cytokine | | J04164 |
| IFITM3 | Human 1-8U gene from interferon-inducible gene family | Cytokine | | X57352 |
| IL8RA | Homo sapiens interleukin 8 receptor alpha (IL8RA) mRNA, complete cds | Cytokine | | L19591 |
| TNFRSF10C | Homo sapiens TRAIL receptor 3 mRNA, complete cds | Cytokine | | AF016267 |
| IL8RB | Homo sapiens interleukin 8 receptor beta (IL8RB) mRNA, complete cds | Cytokine | | L19593 |
| IFNGR2 | Human clone pSK1 interferon gamma receptorCytokine, accessory factor-1 (AF-1) mRNA, complete cds | Signal | Th1Th2, IFNG | U05875 |
| IL1R1 | Human interleukin 1 receptor mRNA, Cytokine, complete cds | Signal | NFkB | M27492 |
| SCYB5 | H.sapiens ENA-78 mRNA; Small inducible cytokine subfamily B (Cys-X-Cys), member 5 (epithelial-derived neutrophil-activating peptide 78) | Cytokine, Signal | NF-kB | X78686 |
| IL2RG | Human mRNA for interleukin 2 receptor gamma chain | Cytokine, Signal | IL4, IL2 | D11086 |
| TNFRSF1A | H.sapiens TNF-R mRNA for tumor necrosisCytokine, factor receptor type 1. | Signal | TNFR1 | X55313 |
| PMS1 | Human DNA mismatch repair protein PMS1 (PMS1 protein homolog 1) | DNArepair | | U13695 |
| NTF5 | Human neurotrophin-4 (NT-4) gene; neurotrophin 5 (neurotrophin 4/5) (NTF5) | GF | | M86528 |
| HSPA6 | Human mRNA for heat shock proteinHSP70B'; Heat shock 70kD protein 6 | hsp | | X51758 |
| HSPA1A | Homo sapiens heat shock 70kD protein 1 (HSPA1A), mRNA; Heat shock 70kD protein 1 | hsp | | NM_005345 |
| HSPA1L | Homo sapiens HSPA1L mRNA for Heat shock protein 70 testis variant, complete cds; Heat shock 70kD protein-like 1 | hsp | | D85730 |
| RALB | Human GTP-binding protein (RALB) mRNA, complete cds. | oncogene | | M35416 |
| VAV2 | VAV2=VAV oncogene homolog [human, fetal brain, mRNA Partial, 2753bp | oncogene | | S76992 |
| RAF1 | Human mRNA for raf oncogene, v-raf-1 murine leukemia viral oncogene homolog 1 | oncogene, Signal | TPO, TCR, PDGF, NGF, MAPK, Insulin, IL6, IL3, IL2, IGF1, FcER, EPO, EGF, CXCR4, BCR | X03484 |
| ARHA | Homo sapiens RHOA proto-oncogene, multi-drug-resistance protein mRNA, 3' end.; Ras homolog gene family, member A | oncogene Signal | D4DGI | L09159 |
| LYN | Human lyn mRNA encoding a tyrosine kinase; V-yes-1 Yamaguchi sarcoma viral related oncogene homolog | oncogene, Signal | FcER, BCR | M16038 |
| BCL3 | Human B-cell lymphoma 3-encoded protein (bcl-3) mRNA, complete cds | oncogene, Signal | NFκB | M31732 |
| SKIL | Human sno oncogene mRNA for snoN protein, ski-related | oncogene, Signal | TGFbeta | X15219 |
| FOS | Homo sapiens v-fos FBJ murine osteosarcoma viral oncogene homolog (FOS), mRNA. | oncogene, Signal, TF | TPO, TCR, PDGF, NGF, Insulin, IL6, IL3, IL2, IGF1, FcER, EPO, EGF, CXCR4, BCR | NM_005252 |
| POLQ | polymerase (DNA-directed), theta | polymerase | | AF043628 |
| SELL | selectin L (lymphocyte adhesion molecule 1) | Selectin | monocyte, neutrophil | M25280 |
| ADCY6 | Homo sapiens adenylyl cyclase type VI mRNA | Signal | Adenylate cyclase-> PKA | AF250226 |
| LMNB1 | Human lamin B mRNA, complete cds, | Signal | TNFR1, FAS | M34458 |
| BIK | Human Bcl-2 interacting killer (BIK) ; NBK apoptotic inducer protein; BP4; BIP 1 | Signal | Mitochondria | U34584 |
| FRAT1 | Homo sapiens frequently rearranged advanced T-cell lymphomas (FRAT1) mRNA | in Signal | Wnt | NM_005479 |
| PRKDC | Homo sapiens DNA-dependent protein kinase catalytic subunit (DNA-PKcs) mRNA | Signal | TNFR1, FAS | U47077 |
| TLR2 | Homo sapiens Toll-like receptor 2 (TLR2) mRNA, complete cds | Signal | NFkB | U88878 |
| PECAM1 | Platelet/endothelial cell adhesion molecule (CD31 antigen), neutrophil; CD31 | Signal | monocyte, neutrophil | M28526 |
| BTK | H.sapiens atk mRNA for agammaglobulinaemia tyrosine kinase | Signal | FcER, CXCR4, BCR | X58957 |
| FCGR2B | Fc fragment of IgG, low affinity IIb, receptor for (CD32) | Signal | B lympho | M28696 |
| MYD88 | Human myleoid differentiation primary response protein MyD88 mRNA, complete cds | Signal | NFkB | U70451 |
| DUSP1 | H.sapiens CL 100 mRNA for protein tyrosine phosphatase, Dual specificity phosphatase 1, MKP1 | Signal | TNFR2, CD40 | X68277 |
| GNB3 | Human guanine nucleotide-binding protein beta-3 subunit mRNA; Guanine nucleotide binding protein (G protein), beta polypeptide 3 | Signal | CXCR4 | M31328 |
| RGS14 | Homo sapiens regulator of G protein signaling RGS 14 mRNA, complete cds. | Signal | | AF037195 |
| GRB2 | Homo sapiens epidermal growth factor receptor-binding protein GRB2 (EGFRBP-GRB2) mRNA sequence | Signal | TPO, TCR, PDGF, NGF, MAPK, Insulin, IL6, IL4, IL3, IL2, IGF1, FcER, EPO, EGF, CXCR4, BCR | M96995 |
| PAK1 | Human p21-activated protein kinase (PAK-alpha; PAK1) | Signal | TNFR1, FAS | U24152 |
| GSK3B | Human protein kinase mRNA; glycogen synthase kinase 3 beta (GSK3 beta); tau kinase subunit; factor A | Signal | Wnt | L33801 |
| RAC1 | Human ras-related C3 botulinum toxin substrate (rac) mRNA ras-related C3 botulinum toxin substrate 1; p21-rac1; ras-like protein TC25 | Signal | BCR | M29870 |
| PAK2 | Human p21-activated protein kinase (PAK-gamma; PAK2); PAK65; S6/H4 kinase | Signal | TNFR1, MAPK, FcER, FAS | U24153 |
| TYK2 | Human tyk2 mRNA for non-receptor protein tyrosine kinase; Tyrosine kinase 2 | Signal | IFNA | X54637 |
| ITGAM | Integrin, alpha M (complement component receptor 3, alpha; also known as CD11b (p 170), macrophage antigen alpha polypeptide) | Signal | monocyte, neutrophil | J03925 |
| APC | adenomatous polyposis coli protein (APC protein); DP2.5 | Signal | Wnt | M74088 |
| ARHGDIB | Human GDP-dissociation inhibitor protein (Ly-GDI) mRNA, D4-GDI | Signal | TNFR1, FAS,D4GDI | L20688 |
| PDCD8 | Homo sapiens apoptosis-inducing factor AIF mRNA, nuclear gene encoding mitochondrial protein; Programmed cell death 8 | Signal | Mitochondria | AF100928 |
| GRO1 | Human mRNA for melanoma growth stimulatory activity (MGSA), groucho | Signal, TF | Wnt | X12510 |
| ISGF3G | Human IFN-responsive transcription factor subunit mRNA; Interferon-stimulated transcription factor 3, gamma (48kD); p48 | Signal, TF | IFNA | M87503 |
| TCF21 | Homo sapiens epicardin mRNA, complete cds. | Signal, TF | Wnt | AF047419 |
| STAT3 | Homo sapiens DNA-binding protein (APRF) mRNA, complete cds | Signal, TF | TPO, PDGF, IL6, EGF | L29277 |
| MAPK14 | Human p38 mitogen activated protein (MAP) kinase mRNA; cytokine suppressive anti-inflammatory drug binding protein (CSAID binding protein; CSBP); MAX-interacting protein 2 (MXI2) | Stress | MAPK, BCR | L35253 |
| SULT1C1 | Human sulfotransferase mRNA family 1C, member 1 (SULT1C1) | sulfotransferase | | U66036 |
| TPST1 | Homo sapiens tyrosylprotein sulfotransferase-1 mRNA | sulfotransferase | | AF038009 |
| DCC | Human tumor suppressor protein DCC precursor; colorectal cancer suppressor | Supressor | | X76132 |
| ERCC2 | H.sapiens ERCC2 gene, exons 1 & 2 (partial). | TF | | X52221 |
| CSF3R | Human granulocyte colony-stimulating factor receptor (G-CSFR-1) mRNA, complete cds | Cytokine | | M59848 |

**Table II:**

| Significantly Downregulated Genes by Stress Load Test | | | | |
|---|---|---|---|---|
| **Symbol** | **Name** | **Category** | **Pathway** | **GenBank** |
| ABCB7 | Homo sapiens ATP binding cassette transporter mRNA, complete cds | ABC transporter | | AF038950 |
| HADH2 | Homo sapiens amyloid beta-peptide binding protein (ERAB) mRNA; Hydroxyacyl-Coenzyme A dehydrogenase, type II | ADH | | U96132 |
| ADH5 | Human alcohol dehydrogenase class III (ADH5) mRNA | ADH | | M29872 |
| ALDH10 | Human microsomal aldehyde dehydrogenase (ALD10) mRNA | ALDH | | U46689 |
| ACE | Homo sapiens dipeptidyl carboxypeptidase 1 (angiotensin I converting enzyme) (ACE) | angiotensin | | NM_000789 |
| DAP3 | Human ionizing radiation resistance conferring protein mRNA; Death associated protein 3 | Appoptosis | | U18321 |
| CSE1L | Human chromosome segregation gene homolog CAS mRNA, Chromosome segregation 1 (yeast homolog)-like | Appoptosis | | U33286 |
| ATP6S14 | ATPase, vacuolar, 14 kD | ATPase | | D49400 |
| ATP1B3 | ATPase, Na+/K+ transporting, beta 3 polypeptide | ATPase | | U51478 |
| KIAA0611 | ATPase type IV, phospholipid-transporting (P-type),(putative) | ATPase | | AB014511 |
| ATP1B3P1 | ATPase, Na+/K+ transporting, beta 3 pseudogene | ATPase | | AF005898 |
| ATP5J2 | ATP synthase, H+ transporting, mitochondrial F0 complex, subunit f, isoform 2 | ATPase | | AF047436 |
| CDC16 | Human CDC16Hs mRNA, complete cds | CellCycle | | U18291 |
| CDKN2C | Homo sapiens cyclin-dependent kinase inhibitor (CDKN2C) mRNA, complete cds.; p18 | CellCycle | | AF041248 |
| CDC10 | hCDC10=CDC10 homolog [human, fetal lung, mRNA, 2314 nt]. | CellCycle | | S72008 |
| CCNA2 | Human mRNA for cyclin A; Cyclin A2 | CellCycle | | X51688 |
| CCNG 1 | Human cyclin G 1 mRNA, complete cds | CellCycle | | U47413 |
| PCNA | Homo sapiens proliferating cell nuclear antigen (PCNA) mRNA | CellCycle, Signal | p53 | NM_002592 |
| IL10RA | Human interleukin-10 receptor mRNA, complete cds | Cytokine | | U00672 |
| IL11RA | H.sapiens mRNA for interleukin-11 receptor | Cytokine | | Z38102 |
| ILF3 | Human nuclear factor NF90 mRNA, complete cds | Cytokine | | U10324 |
| SCYD1 | Human CX3C chemokine precursor, mRNA, alternatively spliced, complete cds | Cytokine | | U84487 |
| TNFSF4 | Human mRNA for glycoprotein 34 (gp34). | Cytokine | | D90224 |
| ADAM17 | Homo sapiens snake venom-like protease (cSVP) mRNA, A disintegrin and metalloproteinase domain 17 (tumor necrosis factor, alpha, converting enzyme) | Cytokine | | U92649 |
| IL13RA2 | Human interleukin-13 receptor mRNA, complete cds | Cytokine | | U70981 |
| TNFSF9 | Human receptor 4-1BB ligand mRNA, complete cds | Cytokine | | U03398 |
| SCYA7 | Homo sapiens mRNA for monocyte chemotactic protein-3 (MCP-3), Small inducible cytokine A7 (monocyte chemotactic protein 3) | Cytokine | | X72308 |
| IL2RB | Human interleukin 2 receptor beta chain (p70-75) mRNA, complete cds | Cytokine, Signal | IL2 | M26062 |
| TRAF5 | Homo sapiens mRNA for TRAF5, complete cds | Cytokine, Signal | | AB000509 |
| EPO | Human mRNA for fetal erythropoietin | Cytokine, Signal | Eryth, EPO | X02157 |
| TRAF3 | Homo sapiens TNF receptor-associated factor 3 (TRAF3) mRNA. | Cytokine, Signal | TNFR2, CD40 | NM_003300 |
| LIPA | Human lysosomal acid lipase/cholesteryl esterase mRNA (cholesterol esterase) | esterase | | M74775 |
| GZMA | Human Hanukah factor serine protease (HuHF) mRNA (cytotoxic T-lymphocyte-associated serine esterase 3) | esterase | | M18737 |
| GJA5 | gap junction protein, alpha 5, 40kD (connexin 40) | Gap-junciton | | L34954 |
| HGF | Human hepatocyte growth factor mRNA (HGF); scatter factor (SF); hepatopoeitin A | GF | | M60718 |
| FGF2 | Human basic fibroblast growth factor (FGF) mRNA (BFGF; FGFB; FGF2) | GF | | M27968 |
| IGFBP7 | prostacyclin-stimulating factor [human, cultured diploid fibroblastcells, mRNA, 1124 nt]. | GF | | S75725 |
| TGFBR1 | Human activin receptor-like kinase (ALK-5) mRNA, complete cds | GF,Signal | TGFbeta | L11695 |
| EEF1A1 | Homo sapiens eukaryotic translation elongation factor 1 alpha 1 (EEF1A1) | glucocorticoids (Cortisol) | | NM_001402 |
| HSPD1 | Heat shock 60kD protein 1 (chaperonin) | hsp | | M34664 |
| HSPCA | Homo sapiens Hsp89-alpha-delta-N mRNA; Heat shock 90kD protein 1, alpha | hsp | | AF028832 |
| HSPE1 | Human chaperonin 10 mRNA; Heat shock 10kD protein 1 | hsp | | U07550 |
| APG-1 | Homo sapiens mRNA for heat shock protein apg-1; Heat shock protein (hsp110 family) | hsp | | AB023421 |
| HSPA10 | Homo sapiens heat shock 70kD protein 10 (HSC71) (HSPA10), mRNA | hsp | | NM_006597 |
| COX10 | Homo sapiens COX10 (yeast) homolog, cytochrome c oxidase assembly protein (heme A: farnesyltransferase) | mitcondria & stress | | NM_001303 |
| COX7A2L | Homo sapiens cytochrome c oxidase subunit VIIa polypeptide 2 like | mitcondria & stress | | NM_004718 |
| COX5A | Homo sapiens cytochrome c oxidase subunit Va | mitcondria & stress | | NM_004255 |
| NRF | Homo sapiens transcription factor NRF | mitcondria & stress | | NM_017544 |
| COX6C | Homo sapiens cytochrome c oxidase subunit VIc (COX6C), nuclear gene encoding mitochondrial protein | mitcondria & stress | | NM_004374 |
| COX7A2 | Homo sapiens cytochrome c oxidase subunit VIIa polypeptide 2 (liver) (COX7A2), nuclear gene encoding mitochondrial protein | mitcondria & stress | | NM_001865 |
| NR1D2 | Homo sapiens mRNA for EAR-1r, complete cds | NR1 | | D16815 |
| NR1H4 | Human farnesol receptor HRR-1 (HRR-1) mRNA, complete cds | NR1(FXR) | | U68233 |
| NR2C2 | Human TR4 orphan receptor mRNA, complete cds | NR2 | | L27586 |
| RAB9 | Human small GTP binding protein Rab9 mRNA, complete cds. | oncogene | | U44103 |
| RAB4 | Homo sapiens GTP-binding protein (RAB4) mRNA, complete cds. | oncogene | | M28211 |
| BMI1 | Human prot-oncogene (BMI-1) mRNA, complete cds | oncogene | | L13689 |
| RAB7L1 | Homo sapiens mRNA for small GTP-binding protein, complete cds | oncogene | | D84488 |
| BCL2 | Human bcl-2 mRNA; apoptosis regulator bcl2 | oncogene, Signal | p53, Mitochondria | M 14745 |
| POLG2 | polymerase (DNA directed), gamma 2, accessory subunit | polymerase | | U94703 |
| POLRMT | polymerase (RNA) mitochondrial (DNA directed) | polymerase | | U75370 |
| POLR2B | polymerase (RNA) II (DNA directed) polypeptide B (140kD) | polymerase | | X63563 |
| POLE | polymerase (DNA directed), epsilon | polymerase | | L09561 |
| RPA40 | RNA polymerase I subunit | polymerase | | AF008442 |
| TCEB1 | transcription elongation factor B (SIII), polypeptide 1 (15kD, elongin C) | polymerase, TF | | L34587 |
| TCEB1L | transcription elongation factor B (SIII), polypeptide 1-like | polymerase, TF | | Z47087 |
| TAF2I | TATA box binding protein (TBP)-associated factor, RNA polymerase II, I, 28kD | polymerase, TF | | D63705 |
| PRKCH | Human protein kinase C-L (PRKCL) mRNA; Protein kinase C, eta | Signal | TPO, TCR, PLC, PDGF, EGF, BCR | MSS284 |
| PPP3CC | calcineurin A catalytic subunit [human, testis, mRNA, 2134 nt]; Protein phosphatase 3 (formerly 2B), catalytic subunit, gamma isoform (calcineurin A gamma) | Signal | TCR, FcER, BCR | S46622 |
| RBBP7 | Human retinoblastoma-binding protein (RbAp46) mRNA, complete cds | Signal | | U35143 |
| IKBKAP | Homo sapiens IkappaB kinase complex associated protein (IKAP) mRNA, complete cds; IKKAP2 | Signal | TNFR2, CD40 | AF044195 |
| CD79B | Human immunoglobulin superfamily member B cell receptor complex cell surface glycoprotein (IGB) mRNA, CD79B | Signal | Bcell, B lympho, BCR | M89957 |
| CD8B1 | Human T lymphocyte surface glycoprotein (CD8-beta) mRNA, complete cds | Signal | TcCell | M36712 |
| PTPN7 | Human mRNA for protein-tyrosine phosphatase; Protein tyrosine phosphatase, non-receptor type 7, HePTP | Signal | TCR | D11327 |
| MST1R | H.sapiens RON mRNA for tyrosine kinase; Macrophage stimulating 1 receptor (c-met-related tyrosine kinase) | Signal | MspRON | X70040 |
| ADCY7 | Homo sapiens adenylate cyclase 7 (ADCY7) | Signal | Adenylate cyclase-> PKA | NM_001114 |
| RBBP4 | Human chromatin assembly factor 1 p48 subunit (CAF1 p48 subunit); retinoblastoma-binding protein 4 | Signal | | X74262 |
| PPP3CB | Human calcineurin A2 mRNA; | Signal | TCR, FcER, BCR | M29551 |
| PRKCN | Homo sapiens EPK2 mRNA for serine/threonine kinase; Protein kinase C, nu | Signal | TPO, TCR, PLC, PDGF, EGF, BCR | AB015982 |
| TRB@ | Human T-cell receptor active beta-chain mRNA, complete cds | Signal | ThCell, TcCell, TCR, Blympho | M12886 |
| AKAP11 | A kinase (PRKA) anchor protein 11 (AKAP11); Homo sapiens mRNA for KIAA0629 protein, partial cds | Signal | Adenylate cyclase-> PKA | AB014529 |
| HINT | Homo sapiens protein kinase C inhibitor (PKCI-1) mRNA, Histidine triad nucleotide-binding protein | Signal | | U51004 |
| ITGB1 | Integrin, beta 1 (fibronectin receptor, beta polypeptide, antigen CD29 includes MDF2, MSK12); | Signal | monocyte | X07979 |
| MAP3K7 | Homo sapiens mitogen-activated protein kinase kinase kinase 7 (MAP3K7), mRNA, TAK1 | Signal | Wnt, TNFR1, TGFbeta, NFkB, MAPK, FAS | NM_003188 |
| HLA-DRA | Human HLA-DR alpha-chain mRNA; Class II MHC alpha | Signal | Th1 Th2, Blympho | K01171 |
| AKAP8 | Homo sapiens A kinase (PRKA) anchor protein 8 (AKAP8) | Signal | Adenylate cyclase-> PKA | NM_005858 |
| SRF | Human serum response factor (SRF) mRNA; Serum response factor (c-fos serum response element-binding transcription factor) | Signal, TF | TF, PDGF, MAPK, Insulin, IL6, IGF1, EGF | J03161 |
| CHST2 | Homo sapiens carbohydrate (N-acetylglucosamine-6-O) sulfotransferase 2 (CHST2) | sulfotransferase | | NM_004267 |
| TPST2 | Homo sapiens tyrosylprotein sulfotransferase-2 mRNA | sulfotransferase | | AF049891 |
| HMG1 | Human mRNA for high mobility group-1 protein (HMG-1). | sulfotransferase | | X12597 |
| ST13 | Homo sapiens putative tumor Supressor ST13 (ST13) mRNA, complete cds | Suppressor | | U 17714 |
| DMBT1 | Homo sapiens mRNA for DMBT1 6 kb transcript variant 1 (DMBT1/6kb.1). | Supressor | | AJ000342 |
| NME2 | Human putative NDP kinase (nm23-H2S) mRNA, complete cds; c-myc purine-binding transcription factor puf | TF | | M36981 |
| TOP2B | H.sapiens TOP2 mRNA for DNA topoisomerase II (partial).; Topoisomerase (DNA) II beta (180kD) | topoiosomerase | | Z15115 |

From the results of the above analysis, it was found that the expression levels immediately before the presentation (A) and one day after the presentation (C) were not significantly different from those in normal times, and that the expression levels immediately after the presentation (B) varied significantly from those in normal times. Actually, genes that show not only a small p-value between immediately before the presentation (A) and immediately after the presentation (B) but also a large p-value between immediately after the presentation (B) and one day after the presentation (C), that is, genes whose expression levels do not return to normal levels one day after loading stress stimulation do not exist, and expression levels of the marker genes vary significantly from immediately before the presentation to immediately after the presentation and return to near normal levels next day.

Accordingly, an attempt was made to narrow down genes that show not only a small p-value (P≤0.25) between immediately before the presentation (A) and immediately after the presentation (B) but also a small p-value (P≤0.25) between immediately after the presentation (B) and one day after the presentation (C) from among the above "stress marker genes". First, t-tests between the two groups of A and B and of B and C were carried out, and 18 genes that showed significant variations both between A and B and between B and C were extracted based on the p-values. Then, the geometric average of the p-value between A and B and the p-value between B and C was determined for each of the genes and ranked, thereby selecting top ten genes (Table III). All of the selected ten genes were genes upregulated by stress load shown in Table I.

**Table III:**

| Especially Useful Marker Genes for Evaluation (Upregulation) | | | | |
|---|---|---|---|---|
| **No.** | **Symbol** | **Name** | **Keyword or Title** | **GenBank** |
| 1 | FRAT1 | Homo sapiens frequently rearranged in advanced T-cell lymphomas (FRAT1) mRNA | Activation of a novel proto-oncogene, Frat1, contributes to progression of mouse T-cell lymphomas | NM_005479 |
| 2 | NFIL3 | Human bZIP protein NF-IL3A (IL3BP1) mRNA, complete cds | can bind and transactivate the human IL-3 promoter | U26173 |
| 3 | SCYB5 | H.sapiens ENA-78 mRNA; Small inducible cytokine subfamily B (Cys-X-Cys), member 5 (epithelial-derived neutrophil-activating peptide 78) | Cloning of a full-length cDNA encoding the neutrophil-activating peptide ENA-78 from human platelets | X78686 |
| 4 | BTK | H.sapiens atk mRNA for agammaglobulinaemia tyrosine kinase | The gene involved in X-linked agammaglobulinaemia is a member of the src family of protein-tyrosine kinases | X58957 |
| 5 | IL8RB | Homo sapiens interleukin 8 receptor beta (IL8RB) mRNA, complete cds | interleukin 8 receptor beta | L19593 |
| 6 | GRO1 | Human mRNA for melanoma growth stimulatory activity (MGSA), groucho | Molecular characterization and chromosomal mapping of melanoma growth stimulatory activity, a growth factor structurally related to beta-thromboglobulin | X12510 |
| 7 | CSF3R | Human granulocyte colony-stimulating factor receptor (G-CSFR-1) mRNA,complete cds | granulocyte colony-stimulating factor receptor | M59818 |
| 8 | LMNB1 | Human lamin B mRNA, complete cds, | In vitro posttranslational modification of lamin B cloned from a human T-cell line | M34458 |
| 9 | HSPA6 | Human mRNA for heat shock protein HSP70B'; Heat shock 70kD protein 6 | heat shock protein; heat shock protein 70; heat shock protein 70B' | X51758 |
| 10 | IFNGR2 | Human clone pSK1 interferon gamma receptor accessory factor-1 (AF-1) mRNA, complete cds | Identification and sequence of an accessory factor required for activation of the human interferon gamma receptor | U05875 |

Since all of the ten genes selected in the above paragraph were genes upregulated by stress load, genes downregulated by stress load were also subjected to the following procedures to narrow down especially useful marker genes. First, genes that showed a small p-value (P≤0.25) between immediately before the presentation (A) and immediately after the presentation (B) or a small p-value (P≤0.25) between immediately after the presentation (B) and one day after the presentation (C) were extracted by t-tests between the two groups of A and B and of B and C. Then, the geometric average of the p-value between A and B and the p-value between B and C was determined for each of the genes and ranked, thereby selecting top eleven genes (Table IV).

**Table IV:**

| Especially Useful Marker Genes for Evaluation (Downregulation) | | | | |
|---|---|---|---|---|
| **No.** | **Symbol** | **Name** | **Keyword or Title** | **GenBank** |
| 1 | CDC16 | Human CDC16Hs mRNA, complete cds | Hanukah factor; T-cell-specific serine protease; natural killer cell-specific serine protease; serine protease | U18291 |
| 6 | GZMA | Human Hanukah factor serine protease (HuHF) mRNA (cytotoxic T-lymphocyte-associated serine esterase 3) | Isolation of a human cDNA for heme A:farnesyltransferase by functional complementation of a yeast cox10 mutant | M18737 |
| 2 | CCNA2 | Human mRNA for cyclin A; Cyclin A2 | interleukin; interleukin 2 receptor beta-chain | X51688 |
| 3 | IL2RB | Human interleukin 2 receptor beta chain (p70-75) mRNA, complete cds | Purification and molecular cloning of prostacyclin-stimulating factor from serum-free conditioned medium of human diploid fibroblast cells | M26062 |
| 4 | TRAF5 | Homo sapiens mRNA for TRAF5, complete cds | Hepatitis B virus integration in a cyclin A gene in a hepatocellular carcinoma | AB000509 |
| 5 | LIPA | Human lysosomal acid lipase/cholesteryl esterase mRNA (cholesterol esterase) | Molecular cloning and chromosomal mapping of the human gene for the testis-specific catalytic subunit of calmodulin-dependent protein phosphatase (calcineurin A) | M74775 |
| 7 | IGFBP7 | prostacyclin-stimulating factor [human, cultured diploid fibroblastcells, mRNA, 1124 nt]. | Structure and expression of a human gene coding for a 71 kd heat shock 'cognate' protein | S75725 |
| 8 | HSPA10 | Homo sapiens heat shock 70kD protein 10 (HSC71) (HSPA10), mRNA | CDC27Hs colocalizes with CDC16Hs to the centrosome and mitotic spindle and is essential for the metaphase to anaphase transition | NM_006597 |
| 9 | COX10 | Homo sapiens COX10 (yeast) homolog, cytochrome c oxidase assembly protein (heme A: farnesyltransferase) | Cloning and characterization of a cDNA encoding the human homolog of tumor necrosis factor receptor-associated factor 5 (TRAF5) | NM_001303 |
| 10 | PPP3CC | calcineurin A catalytic subunit [human, testis, mRNA, 2134 nt]; Protein phosphatase 3 (formerly 2B), catalytic subunit, gamma isoform (calcineurin A gamma) | Dual retinoblastoma-binding proteins with properties related to a negative regulator of ras in yeast | S46622 |
| 11 | RBBP7 | Human retinoblastoma-binding protein (RbAp46) mRNA, complete cds | lysosomal acid lipase/cholesteryl esterase | U35143 |

The especially useful marker genes for evaluation shown in Table III and Table IV included predominantly cytokine related genes and heat shock protein related genes.

### Example 2

As a comparative control of the above example, how the expression levels of stress marker genes vary in response to stimulation such as music appreciation that is free from stress was examined.

### 1. Test method

Thirteen healthy subjects (Sample Nos. 11 to 23) in their twenties to forties were subjected to music appreciation. Right after then, five ml of blood was collected from the elbow vein of each subject, and total RNA was extracted in the same way as in Example 1. The amounts of the total RNA extracted ranged from 6 to 20 micrograms.

Five micrograms of RNA was taken out from the total RNA extracted from each subject and subjected to annealing with an oligo(dT) 24 primer connected to a T7 promotor sequence to synthesize a first strand DNA. Then, with the use of this first strand DNA as a template, a second strand DNA having the T7 promotor sequence was synthesized. Finally, with the use of the second strand DNA as a template, cRNA synthesis was carried out using T7 RNA polymerase. To the synthesized cRNA were annealed random hexamers, followed by subjecting to reverse transcriptase reaction to synthesize a fluorescently labeled cDNA by allowing Cy5-dCTP to be incorporated into the strand. As to the reference sample immediately before the music appreciation (in normal times), cDNA was synthesized using Cy3 as the fluorescent label.

### 2. Results

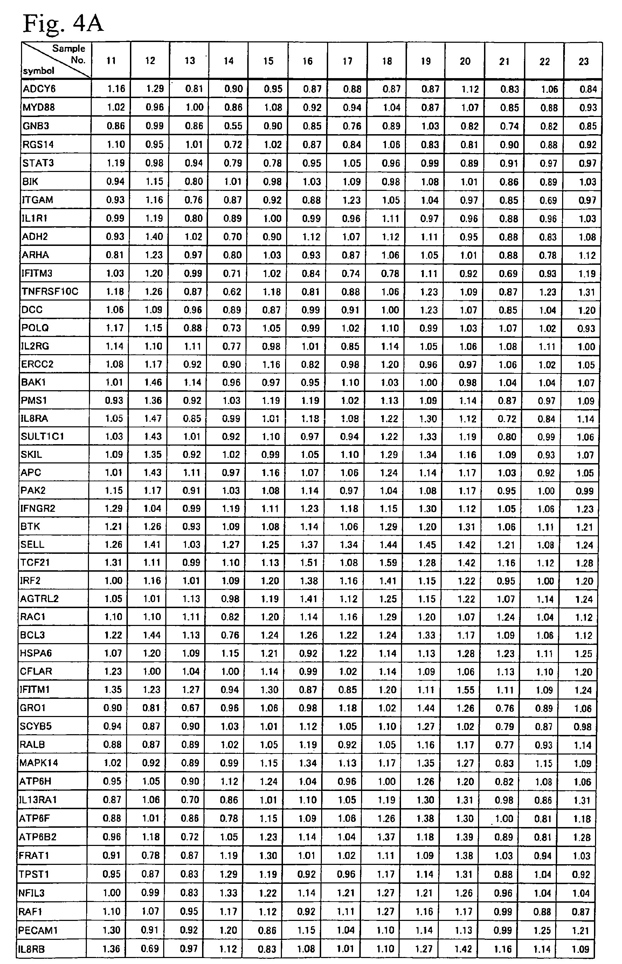

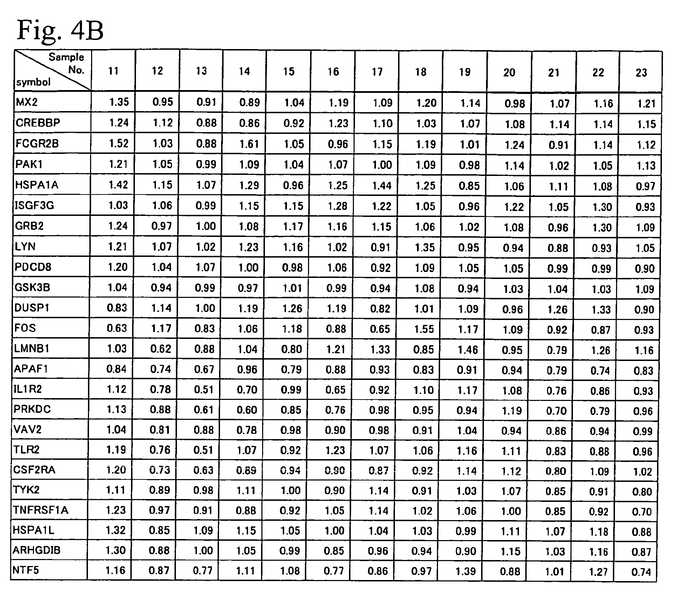

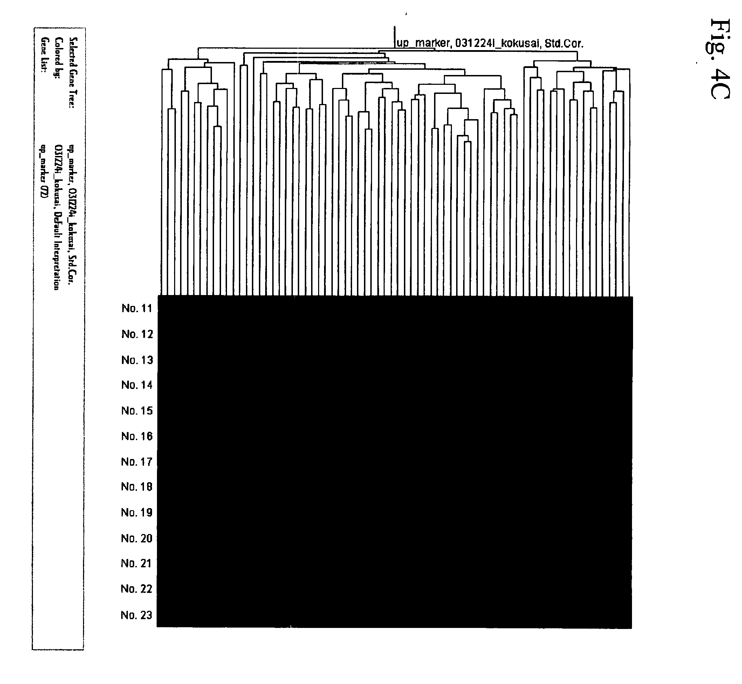

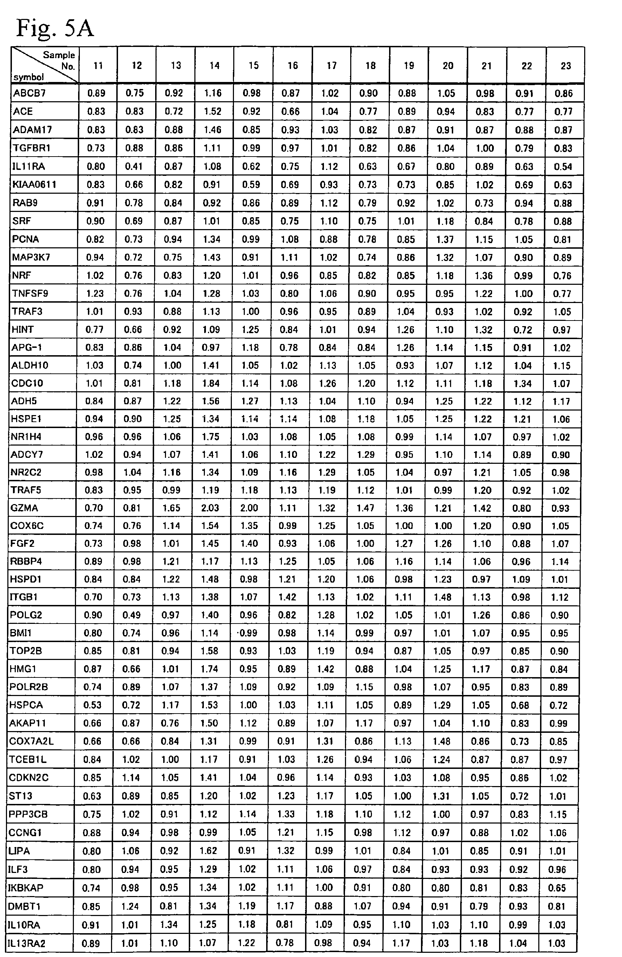

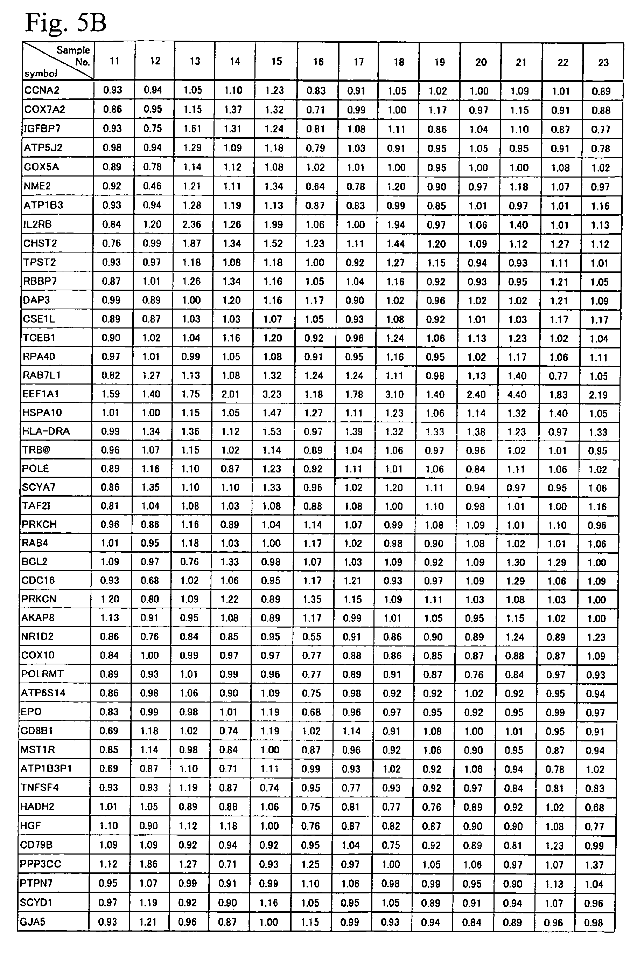

The ratios of fluorescence intensity before and after the music appreciation of the genes listed in Table I and Table II are shown in Figs. 4A, 4B, 5A and 5B. Significant variations were hardly observed in the expression levels of the genes considered to be useful as stress marker genes. Thus, it was confirmed that variations in expression of the genes listed in Table I and Table II occurred characteristically only when undergoing mental stress stimulation.

The method of the present invention can be used as an objective and simple method of stress evaluation not only in individual health management but also in health checkup in office, school, or community, multiphasic health screening, and the like. The present invention contributes greatly to improvement of the nation's mental health in view of preventive medicine.

## Claims

1. A method of stress evaluation comprising:
analyzing expression levels of marker genes including ten genes listed in Table III and/or eleven genes listed in Table IV in mRNA derived from peripheral blood of a subject; and
evaluating stress of the subject based on the analysis results.

2. The method of stress evaluation according to claim 1, wherein the marker genes further include other genes selected from genes listed in Table I and Table II.

3. The method of stress evaluation according to claim 1 or 2, wherein the stress of the subject is evaluated by performing comparative analysis between gene expression profile of a universal RNA sample and gene expression profile of a sample of the subject.

4. The method of stress evaluation according to claim 1 or 2, wherein the stress of the subject is evaluated by performing comparative analysis between gene expression profiles of the same subject in normal times and under stress load.

5. The method of stress evaluation according to any one of claims 1 to 4, wherein expression levels of the genes are analyzed with the use of a solid substrate comprising immobilized DNA selected from DNA chip, DNA array, membrane filter, and capillary.

6. A solid substrate for stress evaluation that is prepared by immobilizing each probe that specifically hybridizes to any one of marker genes selected from genes listed in Table I or Table II, wherein the marker genes include at least ten genes listed in Table III and/or eleven genes listed in Table IV.

7. A system of stress evaluation to perform the method of stress evaluation according to any one of claims 1 to 5, comprising a means that performs comparative analysis between gene expression data of the subject and gene expression data of the subject obtained beforehand in normal times or gene expression data of a universal RNA sample.
